(19) Europäisches Patentamt
European Patent Office
Office européen des brevets

(11) Publication number : 0 506 437 A1

# (12) EUROPEAN PATENT APPLICATION

(21) Application number : 92302680.1

(22) Date of filing : 27.03.92

(51) Int. Cl.$^5$ : **C07D 405/04,** A61K 31/415, C07D 405/14, A61K 31/335

(30) Priority : 27.03.91 GB 9106508

(43) Date of publication of application : 30.09.92 Bulletin 92/40

(84) Designated Contracting States : PT

(71) Applicant : RHONE POULENC RORER LIMITED
Rainham Road
South Dagenham, Essex RM10 XS (GB)

(72) Inventor : Harris, Neil Victor
Rhone-Poulenc Rorer Ltd., Dagenham
Essex Rm10 7XS (GB)
Inventor : Smith, Christopher
Rhone-Poulenc Rorer Ltd., Dagenham
Essex Rm10 7XS (GB)
Inventor : Stuttle, Keith Alfred James
Rhone-Poulenc Rorer Ltd., Dagenham
Essex Rm10 7XS (GB)
Inventor : Walsh, Roger John Aitchison
Rhone-Poulenc Rorer Ltd., Dagenham
Essex Rm10 7XS (GB)
Inventor : Wyman, Barry Mark
Rhone-Poulenc Rorer Ltd., Dagenham
Essex Rm10 7XS (GB)

(74) Representative : Bentham, Stephen
J.A. Kemp & Co. 14 South Square Gray's Inn
London, WC1R 5LX (GB)

## (54) Imidazoles.

(57) Imidazole derivatives of the formula :

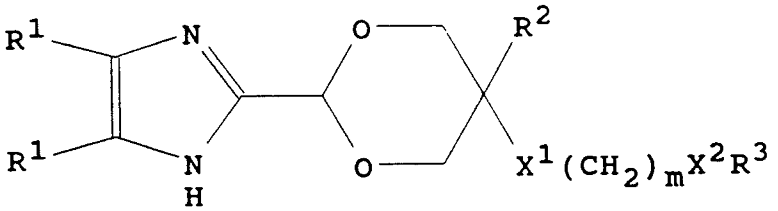

I

wherein $R^1$ represents optionally substituted phenyl, $R^2$ represents alkyl optionally substituted by hydroxy, $X^1$ represents a bond, oxygen, $-S(0)_n-$, $-CH_20-$ or - $CH_2N(R^6)-$, wherein n is 0, 1 or 2 and $R^6$ represents hydrogen, alkyl, acyl alkylsulphonyl or, $-C(X^3)NR^7R^8$, wherein $X^3$ represents oxygen or sulphur and $R^7$ and $R^8$ represent hydrogen, alkyl, or optionally substituted phenyl, m is zero or 1 to 8, $X^2$ represents a bond, oxygen, $-S(0)_p-$ wherein p is 0, 1 or 2, or a group $-C(0)NR^4-$, $-C(S)NR^4-$, $-OC(0)NR^4-$, $-OC(S)NR^4-$, $-NR^4C(0)-$ or $-NR^4C(S)-$, wherein $R^4$ represents hydrogen or alkyl and $R^3$ represents optionally substituted heterocyclyl, or cycloalkyl or, except when $X^1$ and $X^2$ are direct bonds, $R^3$ represents optionally substituted phenyl, or $R^3$ represents amino, alkylamino, benzylamino, benzyl(alkyl)amino, dialkylamino, aminoalkyl, alkylaminoalkyl, benzylaminoalkyl, benzyl(alkyl)aminoalkyl or dialkylaminoalkyl, or a cyclic imino group, and salts thereof possess useful pharmacological properties.

EP 0 506 437 A1

This invention relates to new, therapeutically useful imidazole derivatives, to a process for their production, to pharmaceutical compositions containing them, and to their use in a method of treatment of patients.

The new imidazole derivatives of the present invention are the compounds of the general formula I hereinafter depicted, wherein the symbols $R^1$ are the same or different and each represents an optionally substituted phenyl group, $R^2$ represents an alkyl group, optionally substituted by a hydroxy group, $X^1$ represents a direct bond, an oxygen atom, or a group of the formula $-S(O)_n-$, $-CH_2O-$ or $-CH_2N(R^6)-$, wherein n is 0, 1 or 2 and $R^6$ represents a hydrogen atom, an alkyl group, an acyl group, an alkylsulphonyl group, or a group of the formula $-C(X^3)NR^7R^8$, wherein $X^3$ represents an oxygen or sulphur atom and $R^7$ and $R^8$ are the same or different and each represents a hydrogen atom, an alkyl group, or an optionally substituted phenyl group, m represents zero or an integer from 1 up to about 8, $X^2$ represents a direct bond, an oxygen atom, a group $-S(O)_p-$ wherein p is 0, 1 or 2, n and p preferably being identical, or a group of the formula $-C(O)NR^4-$, $-C(S)NR^4-$, $-OC(O)NR^4-$, $-OC(S)NR^4-$, $-NR^4C(O)-$or $-NR^4C(S)-$, wherein $R^4$ represents a hydrogen atom or an alkyl group, and $R^3$ represents an optionally substituted heterocyclyl group, or a cycloalkyl group containing from about 5 to about 7 carbon atoms or, except when $X^1$ and $X^2$ simultaneously represent direct bonds, $R^3$ represents an optionally substituted phenyl group, or $R^3$ represents an amino, alkylamino, benzylamino, benzyl(alkyl)amino, dialkylamino, aminoalkyl, alkylaminoalkyl, benzylaminoalkyl, benzyl(alkyl)aminoalkyl or dialkylaminoalkyl group, or a cyclic imino group containing in the ring up to about 7 carbon atoms, one or two of which can be in the form of a carbonyl group or groups, and the ring of which may be interrupted by a further nitrogen atom which may carry an alkyl or optionally substituted phenyl group, and their pharmaceutically acceptable salts.

As will be apparent to those skilled in the art, some of the compounds of formula I exhibit optical and/or geometric isomerism. All such forms, and their mixtures, are embraced by the invention.

A preferred class of compounds of general formula I are those wherein the symbols $R^1$ are the same or different and each represents an optionally substituted phenyl group, $R^2$ represents an alkyl group, optionally substituted by a hydroxy group, $X^1$ represents a direct bond, an oxygen atom, or a group of the formula $-S(O)_n-$, $-CH_2O-$ or $-CH_2N(R^6)-$, wherein n is 0, 1 or 2 and $R^6$ represents a hydrogen atom, an alkyl group, an acyl group, an alkylsulphonyl group, or a group of the formula $-C(X^3)NR^7NR^8$, wherein $X^3$ represents an oxygen or sulphur atom and $R^7$ and $R^8$ are the same or different and each represents a hydrogen atom, an alkyl group, or a optionally substituted phenyl group, m represents zero or an integer from 1 up to about 8, $X^2$ represents a direct bond, an oxygen atom, a group $-S(O)_p-$ wherein p is 0, 1 or 2, n and p preferably being identical, or a group of the formula $-C(O)NR^4-$, $-C(S)NR^4-$, $-OC(O)NR^4-$, $-OC(S)NR^4-$, $-NR^4C(O)-$or $-NR^4C(S)-$, wherein $R^4$ represents a hydrogen atom or an alkyl group, and, except when $X^1$ represents an oxygen atom or a group $-CH_2O-$, $R^3$ represents an optionally substituted heterocyclyl group, or $R^3$ represents a cycloalkyl group containing from about 5 to about 7 carbon atoms or, except when $X^1$ and $X^2$ simultaneously represent direct bonds, $R^3$ represents an optionally substituted phenyl group, or $R^3$ represents an amino, alkylamino, benzylamino, benzyl(alkyl) amino, dialkylamino, aminoalkyl, alkylaminoalkyl, benzylaminoalkyl, benzyl(alkyl)aminoalkyl or dialkylaminoalkyl group, or a cyclic imino group containing in the ring up to about 7 carbon atoms, one or two of which can be in the form of a carbonyl group or groups, and the ring of which may be interrupted by a further nitrogen atom which may carry an alkyl or optionally substituted phenyl group, and their pharmaceutically acceptable salts.

A preferred class of compounds of general formula I are those wherein m is other than zero when $X^1$ and $X^2$ simultaneously represent groups $-S(O)_n-$ and $-S(O)_p-$ respectively.

An especially preferred class of compounds of general formula I are those wherein m is other than zero when neither of $X^1$ and $X^2$ represents a direct bond.

Another preferred class of compounds of general formula I are those wherein m is other than zero when $X^2$ is other than a direct bond and simultaneously $R^3$ represents an amino, alkylamino, benzylamino, benzyl-(alkyl)amino or dialkylamino group.

In this specification, unless otherwise specified, alkyl groups and moieties are straight- or branched-chain and preferably contain up to about 6 carbon atoms.

In this specification, unless otherwise specified, optionally substituted phenyl, benzyl and benzoyl groups are optionally substituted by one or more substituents selected from, for example, halogen atoms and alkyl and alkoxy groups.

In this specification, unless otherwise specified, "acyl" groups are, for example, alkanoyl groups or optionally substituted benzoyl groups, or their thio analogues.

In this specification, unless otherwise specified, an optionally substituted heterocyclyl group is preferably a 5-, 6- or 7-membered ring containing 1 or 2 nitrogen atoms and optionally an oxygen or sulphur atom, e.g. a pyridyl, pyrimidinyl, imidazolyl, pyrazolyl or morpholinyl group, optionally substituted by one or more substituents selected from, for example, halogen atoms, amino groups, acylamino groups, alkyl and alkoxy groups, and optionally substituted phenyl groups.

A particular class of compounds of general formula I are those wherein the symbols $R^1$ are the same or different and each represents a phenyl group optionally carrying one or more substituents selected from halogen atoms and alkyl and alkoxy groups containing from 1 to about 3 carbon atoms, $R^2$ represents a straight- or branched-chain alkyl group containing from 1 to about 4 carbon atoms, optionally substituted by a hydroxy group, $X^1$ represents a direct bond, an oxygen atom, or a group $-S(O)_n-$ or $-CH_2O-$wherein n is 0, 1 or 2, m represents zero or an integer from 1 up to about 6, $X^2$ represents a direct bond, an oxygen atom, a group $-S(O)_p-$ wherein p is 0, 1 or 2, n and p preferably being identical, a group of the formula $-C(O)NR^4-$, $-C(S)NR^4-$, $-OC(O)NR^4-$, $-OC(S)NR^4-$, $-NR^4C(O)-$ or $-NR^4C(S)-$, wherein $R^4$ represents a hydrogen atom or a straight- or branched-chain alkyl group containing from 1 to about 4 carbon atoms, and, except when $X^1$ represents an oxygen atom or a group $-CH_2O-$, $R^3$ represents a heterocyclyl group, preferably a 5-, 6- or 7-membered ring containing 1 or 2 nitrogen atoms and optionally an oxygen or sulphur atom, e.g. a pyridyl, pyrimidinyl, imidazolyl or pyrazolyl group, optionally substituted by one or more substituents selected from halogen atoms and alkyl and alkoxy groups containing from 1 to about 4 carbon atoms, or $R^3$ represents a cycloalkyl group containing from about 5 to about 7 carbon atoms or, except when $X^1$ and $X^2$ simultaneously represent direct bonds, $R^3$ represents a phenyl group optionally substituted by one or more substituents selected from halogen atoms and alkyl and alkoxy groups containing from 1 to about 4 carbon atoms, or $R^3$ represents an amino group, a straight- or branched-chain alkylamino group containing from 1 to about 4 carbon atoms, a straight- or branched-chain dialkylamino group containing from 1 to about 4 carbon atoms in each alkyl group, or a cyclic imino group containing in the ring up to about 7 carbon atoms, one or two of which can be in the form of a carbonyl group or groups, and their pharmaceutically acceptable salts.

Particularly important compounds according to the invention include the following:-

A r-2-(4,5-diphenylimidazol-2-yl)-5-methyl-c-5-[(2-(4-morpholinyl)ethoxy)methyl]-1,3-dioxane
B r-2-(4,5-diphenylimidazol-2-yl)-5-methyl-t-5-[(2-(4-morpholinyl)ethoxy)methyl]-1,3-dioxane
C r-2-(4,5-diphenylimidazol-2-yl)-5-methyl-c-5-[(cyclohexanecarboxamido)methyl]-1,3-dioxane
D r-2-(4,5-diphenyl-2-imidazolyl)-c-5-[(cyclohexylaminocarbonyloxy)methyl]-5-methyl-1,3-dioxane
E r-2-(4,5-diphenyl-2-imidazolyl)-5-methyl-t-5-[{2-(2-oxo-1-pyrrolidinyl)ethoxy}methyl]-1,3-dioxane
F r-2-(4,5-diphenyl-2-imidazolyl)-5-methyl-c-5-[{2-(2-oxo-1-pyrrolidinyl)ethoxy}methyl]-1,3-dioxane
G r-2-(4,5-diphenyl-2-imidazolyl)-c-5-[{(2,4-difluorophenyl)aminocarbonyloxy}methyl]-5-methyl-1,3-dioxane
H r-2-(4,5-diphenyl-2-imidazolyl)-c-5-[(3,5-dimethyl-1-pyrazolyl)methyl]-5-methyl-1,3-dioxane
I r-2-(4,5-diphenyl-2-imidazolyl)-5-methyl-c-5-[{(1-methyl-2-imidazolyl)thio}methyl]-1,3-dioxane
J r-2-(4,5-diphenyl-2-imidazolyl)-c-5-[{2-(2,4-difluorophenylaminocarbonyloxy)ethoxy}methyl]-5-methyl-1,3-dioxane
K r-2-(4,5-diphenyl-2-imidazolyl)-c-5-[1-imidazolyl)methyl]-5-methyl-1,3-dioxane
L r-2-(4,5-diphenyl-2-imidazolyl)-t-5-[1-imidazolyl)methyl]-5-methyl-1,3-dioxane
M r-2-(4,5-diphenyl-2-imidazolyl)-5-methyl-c-5-[(4-morpholinyl)methyl]-1,3-dioxane
N r-2-(4,5-diphenyl-2-imidazolyl)-c-5-[{(2,4-dimethoxyphenyl)aminocarbonyloxy}methyl]-5-methyl-1,3-dioxane
O t-5-[2-(2,4-difluorophenylaminocarbonyloxy)-ethoxymethyl]-r-2-(4,5-diphenyl-2-imidazolyl)-5-(hydroxymethyl)-1,3-dioxane
P c-5-[2-(2,4-difluorophenylaminocarbonyloxy)-ethoxymethyl]-r-2-(4,5-diphenyl-2-imidazolyl)-5-(hydroxymethyl)-1,3-dioxane
Q c-5-[{3-(N-benzyl-N-methylamino)propanamido}-methyl]-r-2-(4,5-diphenyl-2-imidazolyl)-5-methyl-1,3-dioxane
R c-5-[4-(3,5-dimethyl-1-pyrazolyl)butyl]-r-2-(4,5-diphenyl-2-imidazolyl)-5-methyl-1,3-dioxane
S t-5-[4-(3,5-dimethyl-1-pyrazolyl)butyl]-r-2-(4,5-diphenyl-2-imidazolyl)-5-methyl-1,3-dioxane
T t-5-[3-(3,5-dimethylpyrazol-1-yl)propyl]-r-(4,5-diphenyl-2-imidazolyl)-5-methyl-1,3-dioxane
U c-5-[5-(3,5-dimethyl-1-pyrazolyl)pentyl]-r-2-(4,5-diphenyl-2-imidazolyl)-5-methyl-1,3-dioxane
V t-5-[5-(3,5-dimethyl-1-pyrazolyl)pentyl]-r-2-(4,5-diphenyl-2-imidazolyl)-5-methyl-1,3-dioxane
W c-5-[4-(3,5-dimethyl-1-pyrazolyl)butyl]-r-2-(4,5-diphenyl-2-imidazolyl)-5-ethyl-1,3-dioxane
X t-5-[4-(3,5-dimethyl-1-pyrazolyl)butyl]-r-2-(4,5-diphenyl-2-imidazolyl)-5-ethyl-1,3-dioxane
Y r-2-(4,5-diphenyl-2-imidazolyl)-5-methyl-c-5-[4-(1-pyrazolyl)butyl]-1,3-dioxane
Z r-2-(4,5-diphenyl-2-imidazolyl)-5-methyl-t-5-[4-(1-pyrazolyl)butyl]-1,3-dioxane
AA r-2-(4,5-diphenyl-2-imidazolyl)-5-methyl-c-5-[4-(N-benzyl-N-methylamino)butyl]-1,3-dioxane
AB r-2-(4,5-diphenyl-2-imidazolyl)-5-methyl-c-5-[4-(4-phenyl-1-piperazinyl)butyl]-1,3-dioxane
AC r-2-(4,5-diphenyl-2-imidazolyl)-5-ethyl-c-5-[4-(N-benzyl-N-methylamino)butyl]-1,3-dioxane
AD r-2-(4,5-diphenyl-2-imidazolyl)-c-5-[4-(N-benzyl-N-methylamino)butyl]-5-propyl-1,3-dioxane
AE r-2-(4,5-diphenyl-2-imidazolyl)-c-5-[4-(4-morpholinyl)butyl]-5-propyl-1,3-dioxane
AF r-2-(4,5-diphenyl-2-imidazolyl)-5-methyl-c-5-[6-(N-benzyl-N-methylamino)hexyl]-1,3-dioxane

AG r-(4,5-diphenyl-2-imidazolyl)-5-ethyl-c-5-[4-(4-morpholinyl)butyl]-1,3-dioxane
AH r-2-(4,5-diphenyl-2-imidazolyl)-5-methyl-c-5-[{2-(N-benzyl-N-methylamino)ethoxy}methyl]-1,3-dioxane
AI r-2-(4,5-diphenyl-2-imidazolyl)-5-methyl-c-5-[{3-(N-benzyl-N-methylamino)propoxy}methyl]-1,3-dioxane
AJ r-2-(4,5-diphenyl-2-imidazolyl)-5-methyl-t-5-[{2-(N-benzyl-N-methylamino)ethoxy}methyl]-1,3-dioxane
AK c-5-[{3-(3,5-dimethyl-1-pyrazolyl)propoxy}-methyl]-r-2-(4,5-diphenyl-2-imidazolyl)-5-(hydroxymethyl)-1,3-dioxane
AL r-2-(4,5-diphenyl-2-imidazolyl)-5-(hydroxymethyl)-c-5-[{2-(N-benzyl-N-methylamino)ethoxy}-methyl]-1,3-dioxane
AM r-2-(4,5-diphenyl-2-imidazolyl)-5-(hydroxymethyl)-t-5-[{2-(N-benzyl-N-methylamino)ethoxy}-methyl]-1,3-dioxane
AN r-2-(4,5-diphenyl-2-imidazolyl)-5-methyl-c-5-[5-(1-piperidinyl)pentyl]-1,3-dioxane
AO r-(4,5-diphenyl-2-imidazolyl)-5-methyl-c-5-[4-(4-morpholinyl)butyl]-1,3-dioxane
AP c-5-[4-(3-amino-5-methyl-1-pyrazolyl)butyl]-r-2-(4,5-diphenyl-2-imidazolyl)-5-methyl-1,3-dioxane
AQ c-5-[4-(3-acetamido-5-methyl-1-pyrazolyl)butyl]-r-2-(4,5-diphenyl-2-imidazolyl)-5-methyl-1,3-dioxane
AR r-2-(4,5-diphenyl-2-imidazolyl)-5-methyl-c-5-[N-{3-(4-morpholinyl)propyl}aminomethyl]-1,3-dioxane
AS r-2-(4,5-diphenyl-2-imidazolyl)-5-methyl-c-5-[N-{3-(1-imidazolyl)propyl}aminomethyl]-1,3-dioxane
AT r-2-(4,5-diphenyl-2-imidazolyl)-5-methyl-c-5-[N-{2-(4-morpholinyl)ethyl}aminomethyl]-1,3-dioxane
AU r-2-(4,5-diphenyl-2-imidazolyl)-5-methyl-c-5-[N-{2-(3,5-dimethyl-1-pyrazolyl)ethyl}aminomethyl]-1,3-dioxane
AV r-2-(4,5-diphenyl-2-imidazolyl)-5-methyl-c-5-[N-{3-(3,5-dimethyl-1-pyrazolyl)propyl}aminomethyl]-1,3-dioxane
AW r-2-(4,5-diphenyl-2-imidazolyl)-5-methyl-c-5-[N-acetyl-N-{2-(4-morpholinyl)ethyl}aminomethyl]-1,3-dioxane
AX r-2-(4,5-diphenyl-2-imidazolyl)-5-methyl-c-5-[N-acetyl-N-{3-(4-morpholinyl)propyl}aminomethyl]-1,3-dioxane
AY r-2-(4,5-diphenyl-2-imidazolyl)-5-methyl-c-5-[N-acetyl-N-{3-(1-imidazolyl)propyl}aminomethyl]-1,3-dioxane
AZ r-2-(4,5-diphenyl-2-imidazolyl)-5-methyl-c-5-[N-acetyl-N-{2-(3,5-dimethyl-1-pyrazolyl)ethyl}aminomethyl]-1,3-dioxane
BA r-2-(4,5-diphenyl-2-imidazolyl)-5-methyl-c-5-[N-acetyl-N-{3-(3,5-dimethyl-1-pyrazolyl)propyl}aminomethylmethyl]-1,3-dioxane
BB r-2-(4,5-diphenyl-2-imidazolyl)-5-methyl-c-5-[N-{3-(4-morpholinyl)propyl}-N-(methylsulphonyl)aminomethylmethyl]-1,3-dioxane
BC r-2-(4,5-diphenyl-2-imidazolyl)-5-methyl-c-5-[N-{3-(3,5-dimethyl-1-pyrazolyl)propyl}-N-(methylsulphonyl) aminomethyl]-1,3-dioxane
BD r-2-(4,5-diphenyl-2-imidazolyl)-5-methyl-c-5-[N-{3-(4-morpholinyl)propyl}-N-(phenylaminocarbonyl)-aminomethyl]-1,3-dioxane
BE r-2-(4,5-diphenyl-2-imidazolyl)-5-methyl-c-5-[N-(methylaminothiocarbonyl)-N-{3-(4-morpholinyl)-propyl} aminomethyl]-1,3-dioxane
BF r-2-(4,5-diphenyl-2-imidazolyl)-5-methyl-c-5-[N-(methylaminocarbonyl)-N-{3-(4-morpholinyl)propyl}-aminomethyl]-1,3-dioxane
BG r-2-(4,5-diphenyl-2-imidazolyl)-5-methyl-c-5-[{2-(N-benzyl-N-methylamino)ethoxy}methyl]-1,3-dioxane dimaleate
BH r-2-(4,5-diphenyl-2-imidazolyl)-5-ethyl-c-5-[4-(1-pyrrolidinyl)butyl]-1,3-dioxane

The letters A to BH are allocated for easy reference later in this specification.

The compounds according to the invention are inhibitors of acyl coenzyme-A:cholesterol-O-acyl transferase (ACAT; EC 2.3.1.26). They are therefore of value as anti-atherosclerotic agents and have utility in the treatment of atherosclerosis, hyperlipidaemia, cholesterol ester storage disease, atheroma in vein grafts, and in the reduction of the atherosclerotic plaque.

They are also inhibitors of the binding of thromboxane $TxA_2$ to its receptors. They are therefore of utility in the treatment of conditions such as thrombosis and myocardial infarction, vasospastic disorders, for example associated with angina, and bronchospasm, for example associated with asthma, or in reperfusion salvage therapy, for example after ischaemic injury.

Compounds within the scope of the present invention exhibit positive pharmacological activities as demonstrated by the following in-vitro and in-vivo tests which are believed to correlate to pharmacological activity in humans and other animals.

In in-vitro tests on human platelet membrane, compounds of the invention produced up to 50% inhibition of the binding of thromboxane $TxA_2$ to its receptors at concentrations down to about 600 nanomolar or less.

In assays performed in-virtro, microsomes, obtained from the livers of rats fed on a diet supplemented with

0.5%w/w cholesterol and 0.25%w/w cholic acid for 7 days, were incubated with radiolabelled oleoyl-CoA in the presence of compounds according to the invention at a concentration of 0.5 or 1 μg/ml. The degree of ACAT inhibition produced was up to 95% or more.

In in-vivo tests, using rats fed on a similar diet to that above and further supplemented by 0.03% w/w of test compound, the compounds according to the invention inhibited increases in plasma cholesterol concentrations, measured after 3 days, relative to control animals fed on the cholesterol supplemented diet without the drug, by up to 75% or more.

The present invention also provides a method for the treatment of a human or animal patient suffering from, or subject to, conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, (for example by reduction of the atherosclerotic plaque), or of an inhibitor of the binding of thromboxane $TxA_2$ to its receptors, such as thrombosis and myocardial infarction, vasospastic disorders, for example associated with angina, and bronchospasm, for example associated with asthma, or in reperfusion salvage therapy, for example after ischaemic injury, which comprises administering to the patient an effective amount of a compound of formula I, or a pharmaceutically acceptable acid addition salt thereof, as hereinbefore defined, to secure an improvement in the condition of the patient.

This invention also provides a compound of formula I, or a pharmaceutically acceptable salt thereof, as hereinbefore defined, for use in a new method of treatment, of the human or animal body, by therapy, of conditions which can be ameliorated by the administration of an inhibitor of acyl coenzyme-A:cholesterol-O-acyl transferase, such as atherosclerosis, hyperlipidaemia, cholesterol ester storage disease and atheroma in vein grafts, (for example by reduction of the atherosclerotic plaque), or of an inhibitor of the binding of thromboxane $TxA_2$ to its receptors, such as thrombosis and myocardial infarction, vasospastic disorders, for example associated with angina, and bronchospasm, for example associated with asthma, or in reperfusion salvage therapy, for example after ischaemic injury.

Compounds of general formula I can be prepared by the application or adaptation of known methods, by which is meant methods used heretofore or described in the literature.

For example, according to a feature of the present invention, compounds of general formula I are prepared by the replacement by hydrogen of the group $R^5$ of a compound of the general formula II hereinafter depicted, wherein $R^1$, $R^2$, $R^3$, $X^1$, $X^2$ and m are as hereinbefore defined and $R^5$ represents a protecting group, for example an optionally substituted benzyl group, by the application or adaptation of known methods. For example, when $R^5$ is an optionally substituted benzyl group it is replaced by a hydrogen atom preferably by treatment with sodium in liquid ammonia, or by catalytic hydrogenation, using a catalyst such as palladium on charcoal.

According to a further feature of the present invention, compounds of general formula I are prepared by the reaction of a compound of the general formula V hereinafter depicted, wherein $R^1$, $R^2$, $X^1$, $X^2$ and m are as hereinbefore defined and $R^9$ represents an alkyl or optionally substituted phenyl group, with a compound of the general formula:-

$$HX^2R^3 \qquad VI$$

wherein $R^3$ and $X^2$ are as hereinbefore defined or an alkali metal salt thereof. This process is particularly suited to the preparation of compounds of formula I wherein $X^1$ represents a direct bond and/or $X^2$ represents a direct bond or an oxygen or sulphur atom, and/or $R^2$ represents an alkyl group.

According to a further feature of the present invention, compounds of general formula I wherein $R^2$ represents a hydroxyalkyl group are prepared from compounds of the general formula VII hereinafter depicted, wherein $R^1$, $R^3$, $X^1$, $X^2$ and m are as hereinbefore defined and $R^{10}$ represents a protected hydroxyalkyl group, for example an alkoxyalkoxyalkyl group, e.g. a 1-ethoxyethoxyalkyl group, by the application or adaptation of known methods for the removal of said protecting group, for example when $R^{10}$ represents an alkoxyalkoxyalkyl group, by hydrolysis in the presence of a dilute mineral acid, e.g. hydrochloric acid.

According to a further feature of the present invention, compounds of general formula I are prepared by the reaction of a compound of the general formula VIII hereinafter depicted, wherein $R^1$ is as hereinbefore defined and $R^{11}$ represents an alkyl, e.g. methyl, group, with a compound of the general formula IX hereinafter depicted, wherein $R^2$, $R^3$, $R^9$, $X^1$, $X^2$ and m are as hereinbefore defined, optionally at elevated temperature.

According to a further feature of the invention, compounds of general formula I are prepared by the interconversion of other compounds of formula I.

For example, compounds of general formula I containing acyloxy or acylamino groups are prepared by the acylation of corresponding compounds containing hydroxy or amino groups by the application or adaptation of known methods of acylation.

As a further example, compounds of formula I wherein $R^6$ represents an alkylsulphonyl group are prepared from corresponding compounds wherein $R^6$ represents a hydrogen atom by the action of the appropriate alkylsulphonyl chloride, preferably in the presence of a base such as triethylamine.

As a further example, compounds of formula I wherein $R^4$ and/or $R^6$ and/or $R^7$ and/or $R^8$ is an alkyl group are prepared from compounds of formula I wherein $R^4$ and/or $R^6$ and/or $R^7$ and/or $R^8$ represents a hydrogen atom by the application or adaptation of known methods of alkylation.

As a further example, compounds of formula I containing a sulphonyl group are prepared by the oxidation of compounds of formula I containing a sulphinyl or thio group and compounds of formula I containing a sulphinyl group are prepared by the oxidation of compounds of formula I containing a thio group using a conventional oxidant, such as a percarboxylic acid (e.g. m-chloroperbenzoic acid), in an inert solvent, such as dichloromethane, at or below room temperature.

By the term "pharmaceutically acceptable salts" as used in this specification is meant acid addition salts the anions of which are relatively innocuous to the animal organism when used in therapeutic doses so that the beneficial pharmaceutical properties of the parent compounds of formula I are not vitiated by side-effects ascribable to those anions.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-β-hydroxynaphthoates, gentisates and di-p-toluoyltartrates.

In this specification reference to compounds of formula I is intended to include reference to their pharmaceutically acceptable salts, where the context so permits.

According to a further feature of the invention, acid addition salts of compounds of formula I are prepared by reaction of the parent compounds of formula I with the appropriate acid, for example in an ethereal medium, e.g. tetrahydrofuran, diethyl ether or a mixture thereof.

As well as being useful in themselves as active compounds, acid addition salts of compounds of formula I are useful for the purposes of purification of the parent compounds of formula I, for example by exploitation of the solubility differences between the salts and the parent compounds, by techniques well known to those skilled in art. The parent compounds of formula I can be regenerated from their acid addition salts by known methods, for example by treatment with an alkali, e.g. aqueous sodium bicarbonate solution or aqueous ammonia solution.

The compounds obtained by the abovementioned processes, including the intermediates, can be purified by the usual physical methods, in particular crystallisation and chromatography, especially to resolve mixtures of enantiomers, for example using a chiral column.

Alternatively, enantiomers can be prepared from the appropriate enantiomeric forms of their intermediates.

The starting materials and intermediates, for example the compounds of formulae II, III, IV, V, VI, VII, VIII and IX hereinafter depicted can also be prepared by the application or adaptation of known methods.

For example, compounds of formula II may be prepared from compounds of the general formula III hereinafter depicted, $R^1$ and $R^5$ being as hereinbefore defined, by the application or adaptation of known methods, for example by reaction with compounds of the general formula:

$$HOCH_2C(R^2)[X^1(CH_2)_mX^2R^3]CH_2OH \qquad IV$$

wherein $X^1$, $X^2$, m, $R^2$ and $R^3$ are as hereinbefore defined. This reaction is generally carried out in an inert organic solvent in the presence of an acidic catalyst. Conveniently, the reaction is carried out in toluene as the solvent, with pyridinium 4-toluenesulphonate as the acidic catalyst, and at the reflux temperature, with azeotropic removal of water.

Alternative known methods of preparation of compounds of formula II by the application or adaptation of known methods are illustrated in the Example and Reference Examples.

For example, a compound of formula III can be reacted with a diol analogous to compounds of formula IV but containing a reactive moiety, and the resulting product can be elaborated, for example by reaction with a compound containing at least the group $R^3$ and another reactive moiety which can react with the former reactive moiety, to give the required compound of formula II. In some cases, protection and subsequent deprotection is desirable. Such reaction sequences will be designed to suit the particular compound of formula II in question, and will be apparent to those skilled in the art.

The following Examples and Reference Examples illustrate the preparation of the compounds according to the invention and illustrate the preparation of the intermediates.

N.M.R. spectra were recorded at 200MHz or 400MHz. Chemical shifts are expressed in ppm relative to tetramethylsilane. Abbreviations have the following significances:- s = singlet, d = doublet, t = triplet, q = quartet, m = multiplet, b = broad.

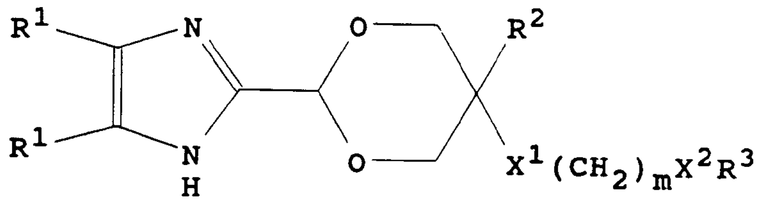

I

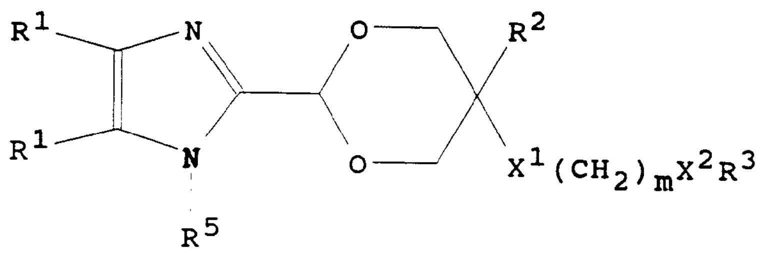

II

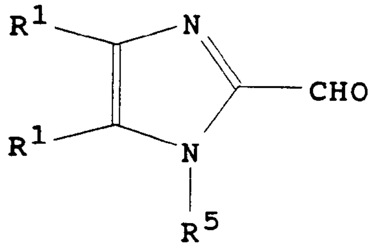

III

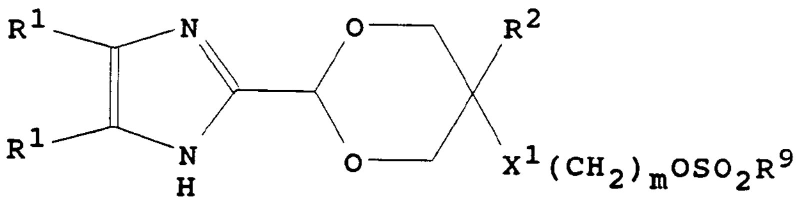

V

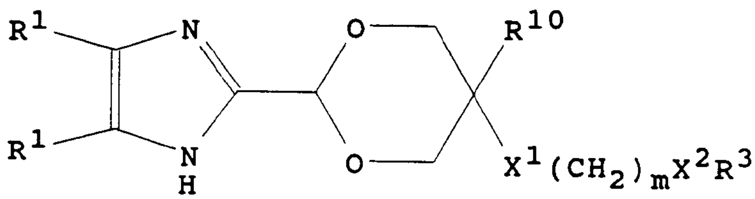

VII

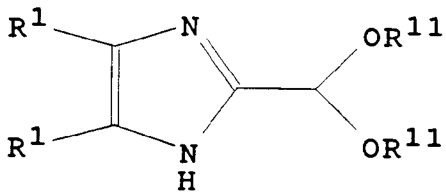

VIII

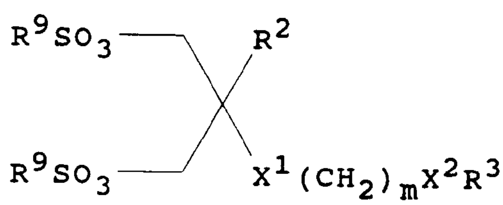

IX

EXAMPLE 1

Compounds A and B

Liquid ammonia (400ml) was condensed into a solution of 2-(1-benzyl-4,5-diphenylimidazol-2-yl)-5-methyl-5-[(2-(4-morpholinyl)ethoxy)methyl]-1,3-dioxane (24.5g of a mixture of cis and trans isomers) in anhydrous tetrahydrofuran (100ml). Small pieces of sodium were added portionwise to this mixture until TLC analysis (eluting with a mixture of methanol and dichloromethane; 1:20v/v) showed that the reaction was complete. The mixture was treated with solid ammonium chloride (20g). After the resulting ammonia had evaporated the residue was partitioned between water (250ml) and ethyl acetate (500ml), the layers were separated, and the organic layer was dried over magnesium sulphate and evaporated. Purification by flash chromatography (eluting with mixtures of methanol and dichloromethane mixtures, 1:19 to 1:9 v/v) gave the following fractions in order of elution:-

(1) the first fraction was crystallised from cyclohexane and a little ethyl acetate to give the cis-isomer, r-2-(4,5-diphenylimidazol-2-yl)-5-methyl-c-5-[(2-(4-morpholinyl)ethoxy)methyl]-1,3-dioxane, Compound A, (8.6g) in the form of a white solid, m.p. 139°C.; [Elemental analysis:- C,70.2;H,7.2;N,9.0%; calculated:- C,70.0;H,7.2;N,9.1%. NMR ($CDCl_3$)- 0.80 (3H,s), 2.51 (4H,t,J = 4Hz), 2.59 (2H,t,J = 6Hz), 3.63 (4H,t,J = 6Hz), 3.64 (2H,s), 3.68 (4H,t,J = 4Hz), 4.05 (2H,d,J = 12Hz), 5.66 (1H,s), 7.2-7.6 (10H,m)];

(2) the second fraction was a mixture of the cis- and trans-isomers; and

(3) the third fraction was triturated with hot cyclohexane to give the trans-isomer, r-2-(4,5-diphenylimidazol-2-yl)-5-methyl-t-5-[(2-(4-morpholinyl)ethoxy)methyl]-1,3-dioxane, Compound B, (2.4g) m.p. 130-131°C.; [Elemental analysis:- C,69.7; H,7.1;N,8.9%. NMR ($CDCl_3$):- 1.28 (3H,s), 2.53 (4H,t, J = 4Hz), 2.69 (2H,t,J = 6Hz), 3.16 (2H,s), 3.55 (2H,t, J = 6Hz), 3.72 (4H,t,J = 4Hz), 3.84 (2H,d,J = 10Hz), 3.97 (2H,d,J = 10Hz), 5.66 (1H,s), 7.2-7.6 (10H,m)].

EXAMPLE 2

Compound C

By proceeding in a manner similar to that described in Example 1, but replacing the 2-(1-benzyl-4,5-diphenylimidazol-2-yl)-5-methyl-5-[(2-(4-morpholinyl)ethoxy)methyl]-1,3-dioxane used as a starting material by the appropriate quantity of r-2-(1-benzyl-4,5-diphenylimidazolyl)-5-methyl-c-5-[(cyclohexanecarboxamido)methyl]-1,3-dioxane, and recrystallising from ethyl acetate, there was prepared r-2-(4,5-diphenylimidazolyl)-5-methyl-c-5-[(cyclohexanecarboxamido)-methyl]-1,3-dioxane in the form of a white solid, m.p. 230°C.; [Elemental analysis:- C,72.1;H,7.4;N,8.9%; calculated for $C_{28}H_{33}N_3O_3$:0.4$H_2O$:- C,72.0;H,7.3;N,9.0%. NMR ($CDCl_3$):- 0.76 (3H,s), 1.1-2.1 (11H,m), 3.65 (2H,d, J = 12Hz), 3.66 (2H,s), 3.91 (2H,d,J = 12Hz), 5.68 (1H,s), 5.84 (1H,bt,J = 6Hz), 7.2-7.6 (10H,m)].

EXAMPLE 3

Compound D

A mixture of 2-(4,5-diphenyl-2-imidazolyl)-5-hydroxymethyl-5-methyl-1,3-dioxane (14.0g) and cyclohexyl isocyanate (5.0g) in toluene (250 ml) was stirred at reflux for 2 days. The mixture was then evaporated to dryness and the residue was subjected to flash chromatography, eluting with a mixture of ethyl acetate and cyclohexane (1:3v/v). Trituration with hot ethyl acetate gave r-2-(4,5-diphenyl-2-imidazolyl)-c-5-[(cyclohexylaminocarbonyloxy)methyl]-5-methyl-1,3-dioxane (9.8g) in the form of a white powder,; m.p. 218°C.; [Elemental analysis:- C,70.9;H,7.1;N,8.8%; calculated:- C,70.7;H,7.0;N,8.8%. NMR ($CDCl_3$):- 0.82 (3H,s), 1.1-2.0 (10H,m), 3.47 (1H,m), 3.65 (2H,d, J = 10Hz), 4.02 (2H,d,J = 10Hz), 5.68 (1H,s), 7.2-7.7 (10H,m)].

EXAMPLE 4

Compounds E, F, G, H, I, J, K, L and M

By carrying out processes similar to those described herein, more especially in the Examples and Reference Examples, there were prepared the following compounds:-

E r-2-(4,5-diphenyl-2-imidazolyl)-5-methyl-t-5-[{2-(2-oxo-1-pyrrolidinyl)ethoxy}methyl]-1,3-dioxane, m.p. 148°C.;

F r-2-(4,5-diphenyl-2-imidazolyl)-5-methyl-c-5-[{2-(2-oxo-1-pyrrolidinyl)ethoxy}methyl]-1,3-dioxane, m.p. 165-166°C.;

G r-2-(4,5-diphenyl-2-imidazolyl)-c-5-[{(2,4-difluorophenyl)aminocarbonyloxy}methyl]-5-methyl-1,3-dioxane, m.p. 183-184°C.;

H r-2-(4,5-diphenyl-2-imidazolyl)-c-5-[(3,5-dimethyl-1-pyrazolyl)methyl]-5-methyl-1,3-dioxane, m.p. 173-174°C.;

I r-2-(4,5-diphenyl-2-imidazolyl)-5-methyl-c-5-[{(1-methyl-2-imidazolyl)thio}methyl]-1,3-dioxane, m.p. 100°C. (ethyl acetate solvate);

J r-2-(4,5-diphenyl-2-imidazolyl)-c-5-[{2-(2,4-difluorophenylaminocarbonyloxy)ethoxy}methyl]-5-methyl-1,3-dioxane, m.p. 143-144°C.;

K r-2-(4,5-diphenyl-2-imidazolyl)-c-5-[1-imidazolyl)methyl]-5-methyl-1,3-dioxane, m.p. 199-200°C.;

L r-2-(4,5-diphenyl-2-imidazolyl)-t-5-[1-imidazolyl)methyl]-5-methyl-1,3-dioxane, m.p. 239°C.; and

M r-2-(4,5-diphenyl-2-imidazolyl)-5-methyl-c-5-[(4-morpholinyl)methyl]-1,3-dioxane, m.p. 205-207°C. (with

decomposition).

EXAMPLE 5

Compounds E, F, H, K, L and Q to AO

By proceeding in a manner similar to that described in Example 1, but using the appropriate 2-(1-benzyl-4,5-diphenylimidazol-2-yl)-1,3-dioxanes as starting materials, there were prepared:-

r-2-(4,5-diphenyl-2-imidazolyl)-5-methyl-t-5-[{2-(2-oxo-1-pyrrolidinyl)ethoxy}methyl]-1,3-dioxane, Compound E, m.p. 148°C.;

r-2-(4,5-diphenyl-2-imidazolyl)-5-methyl-c-5-[{2-(2-oxo-1-pyrrolidinyl)ethoxy}methyl]-1,3-dioxane, Compound F, m.p. 165-166°C.;

r-2-(4,5-diphenyl-2-imidazolyl)-c-5-[(3,5-dimethyl-1-pyrazolyl)methyl]-5-methyl-1,3-dioxane, Compound H, m.p. 173-174°C.;

r-2-(4,5-diphenyl-2-imidazolyl)-c-5-[1-imidazolyl)methyl]-5-methyl-1,3-dioxane, Compound K, m.p. 199-200°C.;

r-2-(4,5-diphenyl-2-imidazolyl)-t-5-[1-imidazolyl)methyl]-5-methyl-1,3-dioxane, Compound L, m.p. 239°C.;

c-5-[{3-(N-benzyl-N-methylamino)propanamido}-methyl]-r-2-(4,5-diphenyl-2-imidazolyl)-5-methyl-1,3-dioxane, Compound Q, white foam, no sharp melting point;

c-5-[4-(3,5-dimethyl-1-pyrazolyl)butyl]-r-2-(4,5-diphenyl-2-imidazolyl)-5-methyl-1,3-dioxane, cyclohexane solvate, Compound R, m.p. 80°C;

t-5-[4-(3,5-dimethyl-1-pyrazolyl)butyl]-r-2-(4,5-diphenyl-2-imidazolyl)-5-methyl-1,3-dioxane, Compound S, m.p. 155-156°C;

t-5-[3-(3,5-dimethylpyrazol-1-yl)propyl]-r-(4,5-diphenyl-2-imidazolyl)-5-methyl-1,3-dioxane, Compound T, m.p. 186-190°C;

c-5-[5-(3,5-dimethyl-1-pyrazolyl)pentyl]-r-2-(4,5-diphenyl-2-imidazolyl)-5-methyl-1,3-dioxane, cyclohexane/water solvate, Compound U, m.p. 98-100°C;

t-5-[5-(3,5-dimethyl-1-pyrazolyl)pentyl]-r-2-(4,5-diphenyl-2-imidazolyl)-5-methyl-1,3-dioxane hydrate, Compound V, m.p. 55-57°C;

c-5-[4-(3,5-dimethyl-1-pyrazolyl)butyl]-r-2-(4,5-diphenyl-2-imidazolyl)-5-ethyl-1,3-dioxane, Compound W, m.p. 124-126°C;

t-5-[4-(3,5-dimethyl-1-pyrazolyl)butyl]-r-2-(4,5-diphenyl-2-imidazolyl)-5-ethyl-1,3-dioxane, Compound X, m.p. 150-151°C;

r-2-(4,5-diphenyl-2-imidazolyl)-5-methyl-c-5-[4-(1-pyrazolyl)butyl]-1,3-dioxane, Compound Y, m.p. 172-173°C;

r-2-(4,5-diphenyl-2-imidazolyl)-5-methyl-t-5-[4-(1-pyrazolyl)butyl]-1,3-dioxane, Compound Z, m.p. 135-136°C;

r-2-(4,5-diphenyl-2-imidazolyl)-5-methyl-c-5-[4-(N-benzyl-N-methylamino)butyl]-1,3-dioxane, Compound AA, m.p. 111-112°C;

r-2-(4,5-diphenyl-2-imidazolyl)-5-methyl-c-5-[4-(4-phenyl-1-piperazinyl)butyl]-1,3-dioxane, Compound AB, m.p. 156-157°C;

r-2-(4,5-diphenyl-2-imidazolyl)-5-ethyl-c-5-[4-(N-benzyl-N-methylamino)butyl]-1,3-dioxane, Compound AC, m.p. 115-117°C;

r-2-(4,5-diphenyl-2-imidazolyl)-c-5-[4-(N-benzyl-N-methylamino)butyl]-5-propyl-1,3-dioxane, Compound AD, m.p. 136-138°C;

r-2-(4,5-diphenyl-2-imidazolyl)-c-5-[4-(4-morpholinyl)butyl]-5-propyl-1,3-dioxane, Compound AE, m.p. 143-145°C;

r-2-(4,5-diphenyl-2-imidazolyl)-5-methyl-c-5-[6-(N-benzyl-N-methylamino)hexyl]-1,3-dioxane, Compound AF, m.p. 105-106°C;

r-(4,5-diphenyl-2-imidazolyl)-5-ethyl-c-5-[4-(4-morpholinyl)butyl]-1,3-dioxane, Compound AG, m.p. 146°C;

r-2-(4,5-diphenyl-2-imidazolyl)-5-methyl-c-5-[{2-(N-benzyl-N-methylamino)ethoxy}methyl]-1,3-dioxane, Compound AH, m.p. 118-120°C;

r-2-(4,5-diphenyl-2-imidazolyl)-5-methyl-c-5-[{3-(N-benzyl-N-methylamino)propoxy}methyl]-1,3-dioxane, Compound AI, m.p. 84-87°C;

r-2-(4,5-diphenyl-2-imidazolyl)-5-methyl-t-5-[{2-(N-benzyl-N-methylamino)ethoxy}methyl]-1,3-dioxane, Compound AJ, m.p. 96°C;

c-5-[{3-(3,5-dimethyl-1-pyrazolyl)propoxy}-methyl]-r-2-(4,5-diphenyl-2-imidazolyl)-5-(hydroxymethyl)-1, 3-dioxane, Compound AK, m.p. 166-168°C;

r-2-(4,5-diphenyl-2-imidazolyl)-5-(hydroxymethyl)-c-5-[{2-(N-benzyl-N-methylamino)ethoxy}-methyl]-1,3-dioxane, cyclohexane solvate, Compound AL, m.p. 60-65°C;

r-2-(4,5-diphenyl-2-imidazolyl)-5-(hydroxymethyl)-t-5-[{2-(N-benzyl-N-methylamino)ethoxy}-methyl]-1,3-dioxane, Compound AM;

r-2-(4,5-diphenyl-2-imidazolyl)-5-methyl-c-5-[5-(1-piperidinyl)pentyl]-1,3-dioxane, Compound AN, m.p. 135-136°C;

r-(4,5-diphenyl-2-imidazolyl)-5-methyl-c-5-[4-(4-morpholinyl)butyl]-1,3-dioxane, cyclohexane solvate, Compound AO, m.p. 93-96°C;

c-5-[4-(3-amino-5-methyl-1-pyrazolyl)butyl]-r-2-(4,5-diphenyl-2-imidazolyl)-5-methyl-1,3-dioxane, Compound AP, non-crystalline foam, no sharp melting point; and

c-5-[4-(3-acetamido-5-methyl-1-pyrazolyl)butyl]-r-2-(4,5-diphenyl-2-imidazolyl)-5-methyl-1,3-dioxane, Compound AQ, non-crystalline foam, no sharp melting point.

EXAMPLE 6

Compounds G, J and N

By proceeding in a manner similar to that described in Example 3, but replacing the cyclohexylisocyanate by the appropriate isocyanates and replacing the 2-(4,5-diphenyl-2-imidazolyl)-5-hydroxymethyl-5-methy1-1,3-dioxane by the appropriate alcohols, there were prepared:-

r-2-(4,5-diphenyl-2-imidazolyl)-c-5-[{(2,4-difluorophenyl)aminocarbonyloxy}methyl]-5-methyl-1,3-dioxane, Compound G, m.p. 183-184°C.;

r-2-(4,5-diphenyl-2-imidazolyl)-c-5-[{2-(2,4-difluorophenylaminocarbonyloxy)ethoxy}methyl]-5-methyl-1,3-dioxane, Compound J, m.p. 143-144°C.; and

r-2-(4,5-diphenyl-2-imidazolyl)-c-5-[{(2,4-dimethoxyphenyl)aminocarbonyloxy}methyl]-5-methyl-1,3-dioxane, Compound N, m.p. 58-60°C.

EXAMPLE 7

Compound I

A stirred solution of 1-methyl-2-mercaptoimidazole (1.26g) in anhydrous dimethylformamide (50ml) was treated with potassium t-butoxide (1.23g). After 15 minutes the mixture was treated with r-(4,5-diphenyl-2-imidazolyl)-5-methyl-c-5-[(toluene-4-sulphonyloxy)-methyl]-1,3-dioxane (5.0g) and stirred at room temperature for 2 hours. The solvent was removed on the rotary evaporator and the residue was partitioned between ethyl acetate (100ml) and water (100ml). The layers were separated and the organic layer was washed with water, dried and evaporated. Crystallisation of the residue from ethyl acetate gave r-2-(4,5-diphenyl-2-imidazolyl)-5-methyl-c-5-[{(1-methyl-2-imidazolyl)-thio}methyl]-1,3-dioxane, ethyl acetate solvate (0.8g) in the form of a white powder, m.p. 100°C; [Elemental analysis:- C,65.6; H,6.31;N,10.9%; calculated for $C_{25}H_{26}N_4O_2S{:}C_4H_8O_2$:- C,65.5; H,5.9;N,10.5%].

EXAMPLE 8

Compound M

A mixture of r-(4,5-diphenyl-2-imidazolyl)-5-methyl-c-5-[(toluene-4-sulphonyloxymethyl]-1,3-dioxane (2.0g) and morpholine (20ml) in dimethylformamide (20ml) was stirred at reflux for 3 hours. The mixture was concentrated and the residue was subjected to flash chromatography, eluting with a mixture of ethyl acetate and dichloromethane (1:1 v/v), and crystallised from cyclohexane, to give r-2-(4,5-diphenyl-2-imidazolyl)-5-methyl-c-5-[(4-morpholinyl)methyl]-1,3-dioxane (0.9g) in the form of a white solid, m.p. 205-207°C; [Elemental analysis:- C,71.6;H,7.0;N,10.0%; calculated:- C,71.56;H,6.96;N,10.01%].

EXAMPLE 9

Compounds O and P

A mixture of r-2-(4.5-diphenyl-2-imidazolyl)-c-5-(1-ethoxyethoxymethyl)-5-(2-hydromethoxymethyl)-1,3-dioxane (11.2g) and 2,4-difluorophenyl isocyanate (4.3g) in toluene (300ml) was stirred at reflux for 6 hours.

The clear solution was concentrated in vacuo and the residual golden oil was dissolved in a mixture of hydrochloric acid (150ml;1.0M) and tetrahydrofuran (100ml). After standing at room temperature for 30 minutes the mixture was basified by the addition of solid potassium carbonate and extracted with ethyl acetate (250ml). The layers were separated and the organic layer was dried and concentrated. Flash chromatography, eluting with a mixture of ethyl acetate and dichloromethane (3:1 v/v), followed by crystallisation from diethyl ether gave t-5-[2-(2,4-difluorophenylaminocarbonyloxy)ethoxymethyl]-r-2-(4,5-diphenyl-2-imidazolyl)-5-(hydroxymethyl)-1,3-dioxane, Compound O, (2.6g) in the form of a white solid, m.p. 142-143°C. [Elemental analysis:C,64.0;H,5.22;N,7.7%; calculated:- C,63.71:H,5.17; N,7.7%].

By proceeding in a similar manner, but using r-2-(4.5-diphenyl-2-imidazolyl)-t-5-(1-ethoxyethoxymethyl)-5-(2-hydromethoxymethyl)-1,3-dioxanes as the starting materials, there was prepared:- c-5-[2-(2,4-difluorophenylaminocarbonyloxy)ethoxymethyl]-r-2-(4,5-diphenyl-2-imidazolyl)-5-(hydroxymethyl)-1,3-dioxane, Compound P, m.p. 179-180°C.

EXAMPLE 10

Compound AR to AV

A stirred suspension of r-2-(1-benzyl-4,5--diphenyl-2-imidazolyl)-5-methyl-1,3-dioxane-c-5-carboxaldehyde (2.0g) in toluene (50ml) was treated with 4-(3-aminopropyl)morpholine and toluene-4-sulphonic-acid (10mg). The mixture was stirred at reflux under a Dean and Stark water separator for 2.5 hours and concentrated to give a pale green gum (3.2g).

A stirred solution of this gum in ethanol (20ml) was treated portionwise during 40 minutes with powdered sodium borohydride (0.59g). After 3 hours a further quantity of sodium borohydride (0.5g) was added and the mixture was allowed to stand at room temperature overnight. The mixture was evaporated and the resulting residue was suspended in water (100ml) and acidified to pH 6 by treatment with dilute hydrochloric acid. The mixture was extracted with ethyl acetate (50ml), and the organic layer was washed with water (50ml), dried, and concentrated to give a colourless gum (2.4g). Ammonia vapour from liquid ammonia (30ml) was condensed into a solution of this gum (2.4g) in anhydrous tetrahydrofuran (25ml). The mixture was treated portionwise with small pieces of sodium until TLC analysis (dichloromethane and methanol, 4:1) showed that reaction was complete. The mixture was treated with solid ammonium chloride (5g) and the ammonia was allowed to evaporate. The residue was partitioned between water (50ml) and ethyl acetate (75ml), the layers were separated, and the organic layer was dried over magnesium sulphate and evaporated. The resulting brown oil was subjected to flash chromatography, eluting with a mixture of dichloromethane and methanol (4:1 v/v), to give r-2-(4,5-diphenyl-2-imidazolyl)-5-methyl-c-5-[N-{3-(4-morpholinyl)propyl}aminomethyl]-1,3-dioxane (0.33g) Compound AR, in the form of a non-crystalline white foam, no sharp melting point. [Elemental analysis:- C,70.9;H,7.8;N,11.8%].

By proceeding in a similar manner, but replacing the 4-(3-aminopropyl)morpholine used as starting material by the appropriate amines, there were prepared:-

r-2-(4,5-diphenyl-2-imidazolyl)-5-methyl-c-5-[N-{3-(1-imidazolyl)propyl}aminomethyl]-1,3-dioxane, Compound AS, m.p. 178-179°C;

r-2-(4,5-diphenyl-2-imidazolyl)-5-methyl-c-5-[N-{2-(4-morpholinyl)ethyl}aminomethyl]-1,3-dioxane, Compound AT, no sharp melting point;

r-2-(4,5-diphenyl-2-imidazolyl)-5-methyl-c-5-[N-{2-(3,5-dimethyl-1-pyrazolyl)ethyl}aminomethyl]1,3-dioxane, Compound AU, an oil; and

r-2-(4,5-diphenyl-2-imidazolyl)-5-methyl-c-5-[N-{3-(3,5-dimethyl-1-pyrazolyl)propyl}aminomethyl]1,3-dioxane, Compound AV, an oil.

EXAMPLE 11

Compounds AW to BA

A solution of r-2-(4,5-diphenyl-2-imidazolyl)-5-methyl-c-5-[N-{2-(4-morpholinyl)ethyl}aminomethyl]-1,3-dioxane (0.40g) in anhydrous dichloromethane (20ml) was treated with acetic anhydride (0.1ml) and the resulting clear solution was allowed to stand at room temperature for 2.5 hours. The solution was washed with aqueous sodium hydrogen carbonate solution (2x7ml;5%w/v) and water (2x10ml), dried, and evaporated. Flash chromatography, eluting with a mixture of dichloromethane and methanol (19:1 v/v), gave r-2-(4,5-diphenyl-2-imidazolyl)-5-methyl-c-5-[N-acetyl-N-{2-(4-morpholinyl)ethyl}aminomethyl]-1,3-dioxane, Compound AW, (0.27g) in the form of a white powder, m.p. 109°C (with decomposition). [Elemental analysis:-

C,67.1;H,7.3;N,10.4%; calculated:- C,67.25; H,7.25;N,10.82%].

By proceeding in a similar manner, but replacing the r-2-(4,5-diphenyl-2-imidazolyl)-5-methyl-c-5-[N-{2-(4-morpholinyl)ethyl}aminomethyl]-1,3-dioxane by the appropriate starting materials, there were prepared:-

r-2-(4,5-diphenyl-2-imidazolyl)-5-methyl-c-5-[N-acetyl-N-{3-(4-morpholinyl)propyl}aminomethyl]-1,3-dioxane, Compound AX, m.p. 180-181°C;

r-2-(4,5-diphenyl-2-imidazolyl)-5-methyl-c-5-[N-acetyl-N-{3-(1-imidazolyl)propyl}aminomethyl]-1,3-dioxane, Compound AY;

r-2-(4,5-diphenyl-2-imidazolyl)-5-methyl-c-5-[N-acetyl-N-{2-(3,5-dimethyl-1-pyrazolyl)ethyl}aminomethylmethyl]-1,3-dioxane, Compound AZ, a non-crystalline foam; and

r-2-(4,5-diphenyl-2-imidazolyl)-5-methyl-c-5-[N-acetyl-N-{3-(3,5-dimethyl-1-pyrazolyl)propyl}aminomethylmethyl]-1,3-dioxane, Compound BA, a non-crystalline foam.

## EXAMPLE 12

### Compounds BB, BC

A stirred solution of r-2-(4,5-diphenyl-2-imidazolyl)-5-methyl-c-5-[N-{3-(4-morpholinyl)propyl}-aminomethyl]-1,3-dioxane (0.25g) in dichloromethane (15ml) was treated with triethylamine (0.064g) and methanesulphonyl chloride (0.060g). After stirring at room temperature for 2 hours the mixture was washed with water (2x10ml), dried, and evaporated. Flash chromatography, eluting with a mixture of dichloromethane and methanol (19:1 v/v), gave r-2-(4,5-diphenyl-2-imidazolyl)-5-methyl-c-5-[N-{3-(4-morpholinyl)propyl}-N-(methylsulphonyl)aminomethyl]-1,3-dioxane, Compound BB, (0.088g) in the form of a non-crystalline white foam. [Elemental analysis:- C,61.4; H,6.72;N,9.6%; calculated:- C,61.81;H,6.93;N,9.95%].

By proceeding in a similar manner, but replacing the r-2-(4,5-diphenyl-2-imidazolyl)-5-methyl-c-5-[N-{3-(4-morpholinyl)propyl}aminomethyl]-1,3-dioxane by r-2-(4,5-diphenyl-2-imidazolyl)-5-methyl-c-5-[N-{3-(3,5-dimethyl-1-pyrazolyl)propyl}aminomethyl]-1,3-dioxane, there was prepared:-

r-2-(4,5-diphenyl-2-imidazolyl)-5-methyl-c-5-[N-{3-(3,5-dimethyl-1-pyrazolyl)propyl}-N-(methylsulphonyl) aminomethyl]-1,3-dioxane, Compound BC, a non-crystalline foam.

## EXAMPLE 13

### Compounds BD to BF

A mixture of r-2-(4,5-diphenyl-2-imidazolyl)-5-methyl-c-5-[N-[3-(4-morpholinyl)propyl}aminomethyl]-1,3-dioxane (0.40g) and phenyl isocyanate (0.12g) in dichloromethane (20ml) was allowed to stand at room temperature for 48 hours. The residue obtained on concentrating the clear solution was subjected to flash chromatography, eluting with a mixture of dichloromethane and methanol (19:1 v/v), to give r-2-(4,5-diphenyl-2-imidazolyl)-5-methyl-c-5-[N-{3-(4-morpholinyl)propyl}-N-(phenylaminocarbonyl)-aminomethyl]-1,3-dioxane, Compound BD, (0.32g) in the form of an off-white solid, m.p. 204-208°C. [Elemental analysis:-C,69.3; H,6.81;N,11.5%; calculated:- C,69.54;H,6.95;N,11.59%].

By proceeding in a similar manner, but replacing the phenyl isocyanate by the appropriate quantities of methyl isothiocyanate and methyl isocyanate respectively, there were prepared:-

r-2-(4,5-diphenyl-2-imidazolyl)-5-methyl-c-5-[N-(methylaminothiocarbonyl)-N-{3-(4-morpholinyl)-propyl} aminomethyl]-1,3-dioxane, Compound BE, and

r-2-(4,5-diphenyl-2-imidazolyl)-5-methyl-c-5-[N-(methylaminocarbonyl)-N-{3-(4-morpholinyl)propyl}-aminomethyl]-1,3-dioxane, Compound BF.

## EXAMPLE 14

### Compound BG

A solution of r-2-(4,5-diphenyl-2-imidazolyl)-5-methyl-c-5-[(2-(N-benzyl-N-methylamino)ethoxy)methyl]-1,3-dioxane (2.0g) in acetone (50ml) at reflux was treated with a solution of maleic acid (1.25g) in acetone (15ml). After stirring at reflux for 10 minutes, the mixture was filtered hot and the filtrate was concentrated until crystallisation began. The mixture was allowed to stand at room temperature for 1 hour and then the solid was filtered off and washed with a small quantity of acetone, to give r-2-(4,5-diphenyl-2-imidazolyl)-5-methyl-c-5-[{2-(N-benzyl-N-methylamino)ethoxy}methyl]-1,3-dioxane dimaleate (2.0g), in the form of a white powder, m.p. 148-149°C. [Elemental analysis:- C,63.8;H,5.88; N,5.69%; calculated:- C,64.19;H,5.94;N,5.76%].

EXAMPLE 15

Compound BH

A mixture of 2-ethyl-2-[4-(1-pyrrolidinyl)-butyl]-1,3-propane-1,3-diol (9.8 g) and 4-toluene-sulphonic acid (8.14 g) in toluene (300 ml) was stirred at reflux under a Dean and Stark water separator for 30 minutes. The mixture was then allowed to cool to 50°C, the Dean and Stark head was replaced by a distillation head, and the mixture was treated with 2-dimethoxymethyl-4,5-diphenylimidazole (6.3 g). The resulting mixture was distilled very slowly. An initial distillate boiling at 63-66°C was observed, and then the temperature of the distillate slowly rose to 110°C. TLC analysis showed that the reaction was complete at this time. The distillate was discarded, and the residual mixture was cooled to room temperature, diluted with ethyl acetate (500 ml), and washed successively with aqueous potassium carbonate solution (500 ml;10%w/v) and water (500 ml). The organic layer was dried and evaporated, to give an orange oil. This oil was subjected to flash chromatography, eluting with a mixture of dichloromethane and methanol and triethylamine (90:9:1v/v), and crystallised from heptane, to give r-2-(4,5-diphenyl-2-imidazolyl)-5-ethyl-c-5-[4-(1-pyrrolidinyl)butyl]-1,3-dioxane (2.5g), in the form of a white solid, m.p. 142-145°C. [Elemental analysis:C,75.6;H,8.1;N,9.3%; calculated:- C,75.78;H,8.11; N,9.14%].

REFERENCE EXAMPLE 1

A solution of 1-benzyl-4,5-diphenylimidazole (2.0g) in anhydrous tetrahydrofuran (25ml) was flushed with argon and cooled in a bath of solid carbon dioxide and acetone (internal temperature below -70°C). Butyllithium (2.5M solution in hexane; 3.2ml) was added dropwise over 5 minutes, maintaining the internal temperature at less than -60°C. The resulting black solution was stirred in the cooling bath for 10 minutes, then 4-formylmorpholine (1.5g) was added dropwise during 1-2 minutes, again keeping the internal temperature at less than -60°C. The cooling bath was removed and the now almost colourless solution was allowed to warm to room temperature during a period of one hour. The solution was poured into hydrochloric acid (25ml; 2M) and extracted with ethyl acetate (50ml). The layers were separated and the ethyl acetate layer was washed with aqueous sodium bicarbonate solution (25ml; 5%w/v), dried over magnesium sulphate and evaporated. Crystallisation of the light yellow residue from acetone gave 1-benzyl-2-formyl-4,5-diphenylimidazole (1.0g) in the form of off-white microcrystals, m.p. 146-147°C.

REFERENCE EXAMPLE 2

A mixture of 1-benzyl-2-formyl-4,5-diphenylimidazole (119.5g), 2-bromomethyl-2-methyl-1,3-propanediol (129.4g), and pyridinium 4-toluenesulphonate (12g) in toluene (1000ml) was stirred at reflux under a Dean and Stark water separator for 24 hours. After cooling to room temperature, the clear solution was washed thoroughly with water, dried over magnesium sulphate and evaporated. Crystallisation of the residue from 2-propanol gave a 3:1 mixture of r-2-(1-benzyl-4,5-diphenylimidazol-2-yl)-c-5-bromomethyl-5-methyl-1,3-dioxane and r-2-(1-benzyl-4,5-diphenylimidazol-2-yl)-t-5-bromomethyl-5-methyl-1,3-dioxane (114.9g), m.p. 148-149°C.

REFERENCE EXAMPLE 3

4-(2-Hydroxyethyl)morpholine (14.5g) was added in small portions during 15 minutes to a stirred suspension of sodium hydride (4.4g of a 60% dispersion in oil) in anhydrous dimethylformamide (250ml). After stirring at room temperature for 1 hour 2-(1-benzyl-4,5-diphenyl-imidazol-2-yl)-5-bromomethyl-5-methyl-1,3-dioxane (3:1 mixture of isomers; 27.9g) was added and the resulting mixture was stirred at reflux for 40 minutes. After cooling to room temperature dimethylformamide was removed on the rotary evaporator and the residue was partitioned between water (250ml) and ethyl acetate (500ml). The layers were separated and the organic layer was washed with water (2x250ml), dried over magnesium sulphate and evaporated.
Crystallisation of the residue from cyclohexane gave 2-(1-benzyl-4,5-diphenylimidazol-2-yl)-5-methyl-5-[(2-(4-morpholinyl)-ethoxy)methyl]-1,3-dioxane in the form of a mixture of cis and trans isomers in the ratio 2.7:1 (24.5g), m.p. 98-99°C.

REFERENCE EXAMPLE 4

Phthalimide (1.8g) was added in small portions to a stirred suspension of sodium hydride (0.5g of a 60% dispersion in oil) in anhydrous dimethylformamide (50ml) at room temperature. After stirring for 30 minutes 2-(1-benzyl-4,5-diphenylimidazol-2-yl)-5-bromomethyl-5-methyl-1,3-dioxane (5.0g) was added and the mixture was stirred at reflux for 2 hours. After cooling to room temperature the solvent was removed on the rotary evaporator and the residue was stirred with water (200ml) for 1 hour. The product was filtered off, washed with water, sucked as dry as possible, and crystallised from a mixture of ethanol and 2-ethoxyethanol, to give 2-(1-benzyl-4,5-diphenyl-2-imidazolyl)-5-[(N-phthalimido)methyl]-5-methyl-1,3-dioxane (2.6g), m.p. 193-196°C.

REFERENCE EXAMPLE 5

A mixture of 2-(1-benzyl-4,5-diphenyl-2-imidazolyl)-5-[(N-phthalimido)methyl]-5-methyl-1,3-dioxane (28.4g) and hydrazine hydrate (3.7g) in ethanol (500ml) was stirred at reflux for 5 hours and then allowed to stand at 0°C. overnight. The mixture was filtered and the residue was washed with a little fresh ethanol. Evaporation of the combined filtrates gave a white solid which was shaken with a mixture of dilute hydrochloric acid (1.0M;200ml) and ethyl acetate (100ml) for 10 minutes. After filtration through filter-aid the layers were separated and the aqueous layer was basified with solid potassium carbonate. The resulting thick white precipitate was collected by filtration and sucked as dry as possible. The residue was taken up in tetrahydrofuran (1 litre) and this solution was washed with saturated aqueous ammonium chloride solution (200ml) and evaporated to dryness. The residue was dried by azeotroping in toluene and then triturated with cyclohexane to give 2-(1-benzyl-4,5-diphenyl-2-imidazolyl)-5-(aminomethyl)-5-methyl-1,3-dioxane (16.5g), a mixture of cis and trans isomers in the approximate ration of 2:1.

REFERENCE EXAMPLE 6

Cyclohexanecarbonyl chloride (1.5g) was added to a stirred suspension of 2-(1-benzyl-4,5-diphenyl-2-imidazolyl)-5-(aminomethyl)-5-methyl-1,3-dioxane (4.4g) and pyridine (1.6g) in dichloromethane (200ml) at room temperature. After stirring for 4 hours a further quantity of cyclohexanecarbonyl chloride (0.7g) was added and the mixture was allowed to stand overnight. The mixture was washed successively with hydrochloric acid (100ml;1.0M) and aqueous sodium bicarbonate solution (100ml;5%w/v), dried (magnesium sulphate), and evaporated to give a brown gum. This mixture was subjected to flash chromatography:-

(1) elution with a mixture of ethyl acetate and cyclohexane (1:1v/v), gave r-2-(1-benzyl-4,5-diphenylimidazolyl)-5-methyl-c-5-[(cyclohexanecarboxamido)-methyl]-1,3-dioxane (2.7g) in the form of a white powder; followed by

(2) elution with ethyl acetate, which gave r-2-(1-benzyl-4,5-diphenylimidazolyl)-5-methyl-t-5-[(cyclohexanecarboxamido)methyl]-1,3-dioxane (0.9g).

REFERENCE EXAMPLE 7

Liquid ammonia (ca.400ml) was condensed into a solution of r-2-(1-benzyl-4,5-diphenylimidazol-2-yl)-c-5-hydroxymethyl-5-methyl-1,3-dioxane (4.9g) in anhydrous tetrahydrofuran (50ml). The mixture was carefully treated with small pieces of sodium until TLC analysis (ethyl acetate) showed that reaction was complete. The mixture was then treated with solid ammonium chloride (10g). After the ammonia had evaporated the mixture was treated with saturated aqueous ammonium chloride solution (100ml) and the mixture was extracted with ethyl acetate (3x100ml). The combined extracts were washed with water (3x100ml), dried over magnesium sulphate and evaporated. Crystallisation of the residue from cyclohexane gave r-2-(4,5-diphenylimidazol-2-yl)-c-5-hydroxymethyl-5-methyl-1,3-dioxane (2,9g) in the form of a colourless solid, m.p. 207-209°C.

REFERENCE EXAMPLE 8

A mixture of 1-benzyl-2-formyl-4,5-diphenylimidazole (13.5g), 1,1,1-trishydroxymethylethane (24g) and pyridinium 4-toluenesulphonate (1.0g) in toluene (400ml) was stirred at reflux under a Dean & Stark water trap for 7 hours. After cooling to room temperature the mixture was washed with water (3x200ml), and the organic layer was dried over magnesium sulphate and evaporated, to give a pale yellow residue (14g) which was subjected to flash chromatography on silica gel, eluting with a mixture of ethyl acetate and cyclohexane (1:1v/v), to give, in order of elution:-

recovered 1-benzyl-2-formyl-4,5-diphenylimidazole (1-29);

r-[1-benzyl-2-(4,5-diphenylimidazol-2-yl)]-c-5-hydroxymethyl-5-methyl-1,3-dioxane (5.8g); and

r-[1-benzyl-2-(4,5-diphenylimidazol-2-yl)]-t-5-hydroxymethyl-5-methyl-1,3-dioxane (4.0g).

REFERENCE EXAMPLE 9

By proceeding in a manner similar to that described in Reference Example 3, but replacing the 4-(2-hydroxyethyl)morpholine used as starting material by the appropriate quantities of 2-benzyloxyethanol, 2-(2-oxo-1-pyrrolidinyl)ethanol, 3,5-dimethylpyrazole, imidazole, 2-(N-benzyl-N-methylamino)ethanol and 3-(N-benzyl-N-methylamino)propanol respectively, there were prepared r-2-(1-benzyl-4,5-diphenyl-2-imidazolyl)-5-[(2-benzyloxyethoxy)methyl]-5-methyl-1,3-dioxane, r-2-(1-benzyl-4,5-diphenyl-2-imidazolyl)-5-methyl-5-[{2-(2-oxo-1-pyrrolidinyl)ethoxy}methyl]-1,3-dioxane, r-2-(1-benzyl-4,5-diphenyl-2-imidazolyl)-5-[(3,5-dimethyl-1-pyrazolyl) methyl]-5-methyl-1,3-dioxane, r-2-(1-benzyl-4,5-diphenyl-2-imidazolyl)-5-[(1-imidazolyl)methyl]-5-methyl-1,3-dioxane, r-2-(1-benzyl-4,5-diphenyl-2-imidazolyl)-5-methyl-c-5-[(2-(N-benzyl-N-methylamino)ethoxy)methyl]-1,3-dioxane, r-2-(1-benzyl-4,5-diphenyl-2-imidazolyl)-5-methyl-t-5-[(2-(N-benzyl-N-methylamino)ethoxy)methyl]-1,3-dioxane, r-2-(1-benzyl-4,5-diphenyl-2-imidazolyl)-5-methyl-c-5-[(3-(N-benzyl-N-methylamino)propoxy)methyl]-1,3-dioxane, and r-3-(1-benzyl-4,5-diphenyl-2-imidazolyl)-5-methyl-t-5-[(2-(N-benzyl-N-methylamino)propoxy)-methyl]-1,3-dioxane.

REFERENCE EXAMPLE 10

By proceeding in a manner similar to that described in Reference Example 7, but replacing the r-2-(1-benzyl-4,5-diphenylimidazol-2-yl)-c-5-hydroxymethyl-5-methyl-1,3-dioxane used as starting material by 2-(1-benzyl-4,5-diphenyl-2-imidazolyl)-5-[(2-benzyloxyethoxy)methyl]-5-methyl-1,3-dioxane and subjecting the product to flash chromatography, there were prepared r-2-(4,5-diphenyl-2-imidazolyl)-t-5-[(2-hydroxyethoxy)methyl]-5-methyl-1,3-dioxane and r-2-(4,5-diphenyl-2-imidazolyl)-c-5-[(2-hydroxyethoxy)-methyl]-5-methyl-1,3-dioxane.

REFERENCE EXAMPLE 11

A mixture of r-2-(4,5-diphenyl-2-imidazolyl)-c-5-(hydroxymethyl)-5-methyl-1,3-dioxane (5.6g) and toluene-4-sulphonyl chloride (10g) in pyridine (50ml) and dichloromethane (50ml) was allowed to stand at room temperature overnight. Water (300ml) was added and the organic phase was separated. The aqueous phase was extracted with dichloromethane (2x25ml) and the combined extracts were dried and concentrated. Crystallisation of the residue from ethyl acetate gave r-(4,5-diphenyl-2-imidazolyl)-5-methyl-c-5-[(toluene-4-sulphonyloxy)methyl]-1,3-dioxane (3.3g) in the form of white crystals, m.p. 193-195°C.

REFERENCE EXAMPLE 12

A mixture of 2-(1-benzyl-4,5-diphenyl)-5-(bromomethyl-methyl)-5-(hydroxymethyl)-1,3-dioxane (26.0g), ethyl vinyl ether (100ml), and pyridinium 4-toluenesulphonate (4.0g) in dichloromethane (400ml) and tetrahydrofuran (400ml) was allowed to stand at room temperature overnight. The clear solution was evaporated to dryness and the residue was dissolved in dichloromethane (100ml). This solution was washed with water (2x100ml), dried and evaporated. The residue was filtered through silica gel, eluting with a mixture of diethyl ether and pentane (1:1 v/v), to give, after evaporation, a white foam (28.3g). This foam was added to a solution of the sodium salt of 2-benzyloxyethanol in dimethylformamide (prepared from 9.5g of 2-benzyloxyethanol and 2.5g of 60% sodium hydride in 300ml of dimethylformamide) and the resulting mixture was stirred at reflux for 45 minutes. After cooling to room temperature the solvent was removed by evaporation and the residual oil was dissolved in ethyl acetate (250ml). This solution was washed with water (2x100ml), dried, and evaporated to give a light yellow oil (25.0g). Liquid ammonia (400ml) was condensed into a solution of this product (25.0g) in anhydrous tetrahydrofuran (75ml). Small pieces of sodium were added portionwise to this mixture until TLC analysis (ethyl acetate) showed that deprotection was complete. The mixture was treated with solid ammonium chloride (20g). After the ammonia had evaporated the residue was partitioned between water (250ml) and ethyl acetate (500ml), the layers were separated, and the organic layer was dried over magnesium sulphate and evaporated. Flash chromatography, eluting with ethyl acetate and cyclohexane mixtures (3:1 to 1:0 v/v), gave r-2-(4.5-diphenyl-2-imidazolyl)-c-5-[{(1-ethoxy)-ethoxy}methyl]-5-[{(2-hydroxy)ethoxy}methyl]-1,3-dioxane (11.2g) in the form of a go1den oil, and r-2-(4.5-diphenyl-2-imidazolyl)-t-5-[{(1-ethoxy)-ethoxy}methyl]-5-[((2-hydroxy)ethoxy)methyl]-1,3-dioxane (4.1g) in the form of a white solid.

By proceeding in a similar manner, but replacing the 2-benzyloxyethanol by the appropriate quantities of 3-(3,5-dimethyl-1-pyrazolyl)propanol and 2-(N-benzyl-N-methylamino)ethanol alcohol, there were prepared r-2-(1-benzyl-4,5-diphenyl-2-imidazolyl)-c-5-[{3-(3,5-dimethyl-1-pyrazolyl)propoxy}methyl]-5-(hydroxymethyl)-1, 3-dioxane-2-(1-benzyl-4,5-diphenyl-2-imidazolyl)-5-(hydroxymethyl)-c-5-[(2-(N-benzyl-N-methylamino)-ethoxy) methyl]-1,3-dioxane; and r-2-(1-benzyl-4,5-diphenyl-2-imidazolyl)-5-(hydroxymethyl)-t-5-[(2-(N-benzyl-N-methylamino)ethoxy)methyl]-1,3-dioxane.

REFERENCE EXAMPLE 13

A stirred solution of c-5-(aminomethyl)-r-2-(1-benzyl-4,5-diphenyl-2-imidazolyl)-5-methyl-1,3-dioxane (10.0g) in anhydrous dichloromethane (150ml) was treated with triethylamine (3.6g) followed, 10 minutes later, by 3-bromopropanoyl chloride (4.4g). After stirring at room temperature overnight the mixture was poured into saturated aqueous ammonium chloride solution (200ml), the layers were separated, and the organic layer was washed with water (2x100ml), dried and evaporated, to give r-2-(1-benzyl-4,5-diphenyl-2-imidazolyl)-c-5-[(3-bromopropanamido)methyl]-5-methyl-1,3-dioxane (13.1g) in the form of a yellow oil.

A mixture of this oil (4.0 g), N-benzylmethylamine (0.87g), and triethylamine (0.72g) in tetrahydrofuran (100ml) was stirred at reflux for 5 hours. The mixture was evaporated to dryness and the residue was partitioned between ethyl acetate (100 ml) and saturated aqueous sodium hydrogen carbonate solution (100 ml). The layers were separated and the organic layer was dried and evaporated. Flash chromatography, eluting with ethyl acetate, gave r-2-(1-benzyl-4,5-diphenyl-2-imidazolyl)-5-methyl-c-5-[{3-(N-benzyl-N-methylamino)propanamido}methyl]-1,3-dioxane, (1.3g) in the form of a yellow oil.

REFERENCE EXAMPLE 14

3,5-Dimethylpyrazole (14.4g) was added in small portions during 15 minutes at room temperature to a stirred suspension of sodium hydride (6.0g of a 60% suspension in oil) in anhydrous tetrahydrofuran (250ml). After stirring for a further 15 minutes, diethyl (4-bromobutyl)methylmalonate ((50.7g) was added and the mixture was stirred at reflux for 8 hours. The mixture was concentrated in vacuo and the residue was partitioned between diethyl ether (300ml) and water (150ml). The layers were separated and the organic layer was washed with water (150ml), dried and evaporated. The residue was passed down a short column of silica gel, eluting with a mixture of diethyl ether and pentane (1:1v/v), to give diethyl [4-(3,5-dimethyl-1-pyrazolyl)butyl]methylmalonate (28.8g), in the form of a colourless mobile oil.

A solution of this oil (28.8g) in anhydrous tetrahydrofuran (50ml) was added dropwise with stirring at room temperature to a solution of lithium aluminium hydride (110ml of a 1.0M solution in tetrahydrofuran) in anhydrous tetrahydrofuran (150ml) at such a rate that the internal temperature did not exceed 35°C. After standing at room temperature overnight aqueous sodium hydroxide solution (22ml;3%w/v) was added dropwise with ice-cooling. The mixture was stirred at room temperature for 15 minutes and then it was filtered through filter-aid. The filter pad was washed thoroughly with fresh tetrahydrofuran and the combined filtrates were evaporated to dryness. The viscous oily residue was dissolved in toluene (200ml) and this solution was stirred at reflux under a Dean and Stark water separator until no more water was distilled from the mixture. Evaporation gave 2-[4-(3,5-dimethyl-1-pyrazolyl)butyl]-2-methyl-1,3-propanediol (21.2g) in the form of a white hazy oil. This oil was dissolved in toluene (300ml) and treated with 1-benzyl-4,5-diphenylimidazole-2-carboxaldehyde (29.6g) and pyridinium 4-toluenesulphonate (2.0g). This mixture was stirred at reflux under a Dean and Stark water separator for 48 hours. After cooling to room temperature the red solution was diluted with ethyl acetate (500ml), washed with water (2x250ml), dried, and evaporated. The resulting red oil was subjected to flash chromatography, eluting with a mixture of ethyl acetate and cyclohexane (l:l v/v), to give r-2-(1-benzyl-4,5-diphenyl-2-imidazolyl)-c-5-[4-(3,5-dimethyl-1-pyrazolyl)butyl]-5-methyl-1,3-dioxane (13.2g), r-2-(1-benzyl-4,5-diphenyl-2-imidazolyl)-t-5-[4-(3,5-dimethyl-1-pyrazolyl)butyl]-5-methyl-1,3-dioxane (8.9g), and a mixture of these cis and trans isomers (7.4g).

By proceeding in a similar manner, but replacing the 3,5-dimethylpyrazole and diethyl (4-bromobutyl)-methylmalonate by the appropriate starting materials, there were prepared r-2-(1-benzyl-4,5-diphenyl-2-imidazolyl)-t-5-[4-(3,5-dimethyl-1-pyrazolyl)butyl]-5-methyl-1,3-dioxane, r-(1-benzyl-4,5-diphenyl-2-imidazolyl)-c-5-[3-(3,5-dimethylpyrazol-1-yl)propyl]-5-methyl-1,3-dioxane, r-2-(1-benzyl-4,5-diphenyl-2-imidazolyl)-c-5-[5-(3,5-dimethyl-1-pyrazolyl)pentyl]-5-methyl-1,3-dioxane, r-2-(1-benzyl-4,5-diphenyl-2-imidazolyl)-t-5-[5-(3,5-dimethyl-1-pyrazolyl)pentyl]-5-methyl-1,3-dioxane, r-2-(1-benzyl-4,5-diphenyl-2-imidazolyl)-c-5-[4-(3,5-dimethyl-1-pyrazolyl)but-1-yl]-5-ethyl-1,3-dioxane, r-2-(1-benzyl-4,5-diphenyl-2-imidazolyl)-t-5-[4-(3,5-dimethyl-1-pyrazolyl)but-1-yl]-5-ethyl-1,3-dioxane, r-2-(1-benzyl-4,5-diphenyl-2-imidazolyl)-5-methyl-c-5-[4-(1-pyrazolyl)butyl]-1,3-dioxane and r-2-(1-benzyl-4,5-diphenyl-2-imidazolyl)-5-methyl-t-5-[4-(1-pyrazolyl)butyl]-1,3-dioxane.

REFERENCE EXAMPLE 15

A mixture of diethyl (4-bromobutyl)methylmalonate (37.4g) and N-benzyl-N-methylamine (14.7g) in tetrahydrofuran (250ml) was stirred at reflux overnight. Concentration in vacuo gave a cream solid which was partitioned between aqueous potassium carbonate solution (200ml;10%w/v) and ethyl acetate (200ml). The layers were separated and the organic layer was washed with water (150ml), dried, and evaporated. The residue was passed down a short column of silica gel with a mixture of diethyl ether and pentane (1:1v/v) to give diethyl [4-(N-benzyl-N-methyl)butyl]-methylmalonate (23.4g) in the form of a golden mobile oil.

A solution of this oil (23.4g) in anhydrous tetrahydrofuran (50ml) was added dropwise with stirring at room temperature to a suspension of lithium aluminiumhydride (3.6g) in anhydrous tetrahydrofuran (250ml) at such a rate that the internal temperature did not exceed 35°C. After standing at room temperature overnight aqueous sodium hydroxide solution (22ml;3%w/v) was added dropwise with ice-cooling. The mixture was stirred at room temperature for 15 minutes and then it was filtered through filter-aid. The filter pad was washed thoroughly with fresh tetrahydrofuran and the combined filtrates were evaporated to dryness. The viscous oily residue was dissolved in toluene (200ml) and this solution was stirred at reflux under a Dean and Stark water separator until no more water was distilled from the mixture. Evaporation gave 2-methyl-2-[4-(N-benzyl-N-methyl)butyl]-1,3-propanediol (17.6g), in the form of a white hazy oil.

This oil was dissolved in toluene (300ml) and treated with 1-benzyl-4,5-diphenylimidazole-2-carboxaldehyde (22.6g) and toluene-4-sulphonic acid (12.7g). This mixture was stirred at reflux under a Dean and Stark water separator for 24 hours. After cooling to room temperature the red solution was partitioned between aqueous potassium carbonate solution (250ml;10%w/v) and ethyl acetate (500ml). The layers were separated and the organic layer was washed with water (2x250ml), dried, and evaporated. The resulting dark oil was subjected to flash chromatography (eluting with ethyl acetate) to give r-2-(1-benzyl-4,5-diphenyl-2-imidazolyl)-5-methyl-c-5-[4-(N-benzyl-N-methyl)-butyl]-1,3-dioxane (16.5g), r-2-(1-benzyl-4,5-diphenyl-2-imidazolyl)-5-methyl-t-5-[4-(N-benzyl-N-methyl)-butyl]-1,3-dioxane (5.9g), m.p. 120°C [Elemental analysis:- C,79.6;H,7.3;N,7.0%; calculated:- C,79.96; H,7.40;N,7.17%], and a mixture of these cis and trans isomers (5.0g).

By proceeding in a similar manner, but replacing the diethyl (4-bromobutyl)methylmalonate and N-benzyl-N-methylamine by the appropriate starting materials, there were prepared r-2-(1-benzyl-4,5-diphenyl-2-imidazolyl)-5-methyl-c-5-[4-(4-phenyl-1-piperazinyl)butyl]-1,3-dioxane, r-(1-benzyl-4,5-diphenyl-2-imidazolyl)-5-methyl-c-5-[4-(4-morpholinyl)butyl]-1,3-dioxane, r-2-(1-benzyl-4,5-diphenyl-2-imidazolyl)-5-ethyl-c-5-[4-(N-benzyl-N-methylamino)butyl]-1,3-dioxane, r-2-(1-benzyl-4,5-diphenyl-2-imidazolyl)-c-5-[4-(N-benzyl-N-methylamino) butyl]-5-propyl-1,3-dioxane, r-2-(1-benzyl-4,5-diphenyl-2-imidazolyl)-c-5-[4-(4-morpholinyl)butyl]-5-propyl-1,3-dioxane, r-2-(1-benzyl-4,5-diphenyl-2-imidazolyl]-5-methyl-c-5-[6-(N-benzyl-N-methylamino)hexyl]-1,3-dioxane and r-(1-benzyl-4,5-diphenyl-2-imidazolyl)-5-ethyl-c-5-[4-(4-morpholinyl)butyl]-1,3-dioxane.

REFERENCE EXAMPLE 16

Piperidine (29.8g) was added dropwise with stirring to an ice-cooled solution of 5-bromopentanoyl chloride (35g) in anhydrous dichloromethane (250ml) at such a rate that the internal temperature was maintained at or below 5°C. The mixture was stirred in the ice-bath for 2 hours and then it was allowed to stand at room temperature overnight. The mixture was washed successively with water (200ml), hydrochloric acid (200ml;1.0M), and aqueous sodium hydrogen carbonate solution (200ml;5%w/v), dried, and evaporated. Distillation of the residue gave 1-(5-bromopentanoyl)-piperidine (25.4g) in the form of a colourless mobile oil, b.p. 120-121°C (0.6mbar).

Diethyl methylmalonate (17.6g) was added dropwise with stirring to a stirred suspension of sodium hydride (4.1g of a 60% dispersion in oil) in anhydroustetrahydrofuran (250ml). After stirring at room temperature for 30 minutes 1-(5-bromopentanoyl)piperidine (25.4g) was added in one portion and the mixture was stirred at reflux for 2 hours. After evaporation to dryness the residue was partitioned between diethyl ether (200ml) and water (200ml), the layers were separated, and the organic layer was dried and concentrated. Distillation of the residue gave diethyl [4-(1-piperidinylcarbonyl)butyl]methylmalonate (25.0g) in the form of a colourless mobile oil, b.p. 160°C (0.6mbar).

A solution of this oil (25.0g) in anhydrous tetrahydrofuran (50ml) was added dropwise with stirring at room temperature to a suspension of lithium aluminium hydride (5.7g) in anhydrous tetrahydrofuran (400ml) at such a rate that the internal temperature did not exceed 50°C. The mixture was stirred at reflux for 3 hours and then it was allowed to stand at room temperature overnight. Aqueous sodium hydroxide solution (40ml;3%w/v) was added dropwise with ice-cooling. The mixture was stirred at room temperature for 1 hour and then it was filtered through filter-aid. The filter pad was washed thoroughly with tetrahydrofuran and the combined filtrates were evaporated to dryness, to give 2-methyl-2-[4-(1-piperidinylcarbonyl)butyl]-1,3-propanediol, in the form of a viscous colourless oil.

This oil was treated with 1-benzyl-4,5-diphenylimidazole-2-carboxaldehyde (25.4g) and toluene-4-sulphonic acid (14.0g) in toluene (250ml) and the resulting mixture was stirred at reflux under a Dean and Stark water separator overnight. After cooling to room temperature the red solution was partitioned between aqueous

potassium carbonate solution (200ml; 10%w/v) and ethyl acetate (400ml). The organic layer was washed with water (2x150ml), dried, and evaporated. The resulting dark oil was subjected to flash chromatography, eluting with a mixture of dichloromethane and methanol (19:1 v/v), to give r-2-(1-benzyl-4,5-diphenyl-2-imidazolyl)-5-methyl-c-5-[5-(1-piperidinyl)pentyl]-1,3-dioxane (17.5g) in the form of a white powder, m.p. 74-79°C. [Elemental analysis:- C,78.4;H,8.0;N,7.3%; calculated:- C,78.8; H,8.04;N,7.45%].

By proceeding in a similar manner, but replacing the piperidine and 5-bromopentanoyl chloride by the appropriate quantities of morpholine and 4-bromobutanoyl chloride, there was prepared r-(1-benzyl-4,5-diphenyl-2-imidazolyl)-5-methyl-c-5-[4-(4-morpholinyl)butyl]-1,3-dioxane.

REFERENCE EXAMPLE 17

Pyridinium chlorochromate (52.0g) was added in one portion to a stirred suspension of r-2-(1-benzyl-4,5-diphenyl-2-imidazolyl)-c-5-(hydroxymethyl)-5-methyl-1,3-dioxane (50.0g) in dichloromethane (1000ml). The mixture was stirred at room temperature and further aliquots of pyridinium chlorochromate (4x7.0g) were added at 2 hourly intervals. The mixture was stirred at room temperature for 3 days, further aliquots of pyridinium chlorochromate (8.0g) being added at 24 hour intervals. The mixture was filtered through filter-aid and the filtrate was concentrated in vacuo. The resulting black gum was dissolved in tetrahydrofuran (500ml) and filtered twice, through two pads of alumina, and evaporated to dryness, to give r-2-(1-benzyl-4.5-diphenyl-2-imidazolyl)-5-methyl-1,3-dioxane-c-5-carboxaldehyde (25.7g) in the form of an off-white powder.

REFERENCE EXAMPLE 18

A mixture of 1-benzyl-4,5-diphenyl-2-imidazole-carboxaldehyde (30g), 2-(4-chlorobutyl)-2-methyl-1,3-propanediol (16g), and pyridinium 4-toluenesulphonate (2g) in toluene (400ml) was stirred at reflux overnight under a Dean and Stark water separator. After cooling to room temperature the mixture was washed with saturated aqueous sodium hydrogen carbonate solution (150ml), then with water (200ml), dried, and evaporated. Flash chromatography, eluting with a mixture of diethyl ether and cyclohexane (1:3 v/v), gave 2-(1-benzyl-4,5-diphenyl-2-imidazolyl)-5-(4-chlorobutyl)-5-methyl-1,3-dioxane (24.8g) in the form of a white foam. A solution of 3-amino-5-methylpyrazole (1.0g) in anhydrous dimethylformamide (50ml) was treated with potassium tert-butoxide (1.0g) and the mixture was stirred at room temperature under nitrogen for 30 minutes. The resulting dark green solution was treated dropwise during 10 minutes with a solution of 2-(1-benzyl-4,5-diphenyl-2-imidazolyl)-5-(4-chlorobutyl)-5-methyl-1,3-dioxane (2.0g) in anhydrous dimethylformamide (20ml). The mixture was stirred at room temperature for 4 hours and then allowed to stand for 2 days. The mixture was poured onto water (500ml) and taken to pH 7 by treatment with dilute hydrochloric acid. The product was extracted with ethyl acetate (3x100ml), and the combined extracts were washed with water, dried, and evaporated. The resulting red gum was subjected to flash chromatography, eluting with a mixture of methanol and dichloromethane (1:9 v/v), and HPLC, to give c-5-[4-(3-amino-5-methyl-1-pyrazolyl)-butyl]-r-2-(1-benzyl-4,5-diphenyl-2-imidazolyl)-5-methyl-1,3-dioxane (0.52g), in the form of a fawn gum.

By proceeding in a similar manner, but replacing the 3-amino-5-methyl-pyrazole by 3-acetamido-5-methyl-pyrazole, there was prepared c-5-[4-(3-acetamido-5-methyl-1-pyrazolyl)butyl]-r-2-(1-benzyl-4,5-diphenyl-2-imidazolyl)-5-methyl-1,3-dioxane.

REFERENCE EXAMPLE 19

A mixture of 1-benzyl-4,5-diphenylimidazole-2-carboxaldehyde (40g) and 4-toluenesulphonic acid (4g) in anhydrous methanol (1000ml) was stirred at reflux for 48 hours. The residue obtained on concentration was dissolved in ethyl acetate (500ml), and this solution was washed with aqueous sodium bicarbonate solution (2x800ml;5%) and water (2x800ml), dried, and evaporated. Crystallisation from cyclohexane gave 1-benzyl-2-dimethoxymethyl-4,5-diphenylimidazole (27.6g).

By proceeding in a manner similar to that described in Example 1 this compound was debenzylated, to give 2-dimethoxymethyl-4,5-diphenylimidazole, in the form of a white solid, m.p. 168-70°C.

The present invention also includes within its scope pharmaceutical formulations which comprise at least one of the compounds of formula I or a pharmaceutically acceptable salt thereof in association with a pharmaceutically acceptable carrier or coating. In clinical practice the compounds of the present invention may be administered parenterally, rectally or orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Preparations according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving, wetting, emulsifying and dispersing agents. They may be sterilised, for example, by filtration through a bacteria-retaining filter, by incorporation in the compositions of sterilising agents, by irradiation or by heating. They may also be manufactured in the form of sterile solid compositions, which can be dissolved in sterile water or some other sterile injectable medium immediately before use.

Solid compositions for rectal administration include suppositories formulated in accordance with known methods and containing at least one compound of formula (I).

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from about 0.5 to about 70, preferably about 1 to about 10, mg/kg body weight per day by oral administration.

The following Composition Example illustrates pharmaceutical compositions according to the present invention.

COMPOSITION EXAMPLE 1

No. 2 size gelatin capsules each containing:-

| | |
|---|---|
| r-2-(4,5-diphenylimidazol-2-yl)-5-methyl-c-5-[(2-(4-morpholinyl)-ethoxy)methyl]-1,3-dioxane | 20 mg |
| lactose | 100 mg |
| starch | 60 mg |
| dextrin | 40 mg |
| magnesium stearate | 1 mg |

were prepared in accordance with the usual procedure.

Similarly, capsules containing any other of the compounds B to BH, or their pharmaceutically acceptable salts, can be prepared.

**Claims**

1. An imidazole derivative of the general formula:

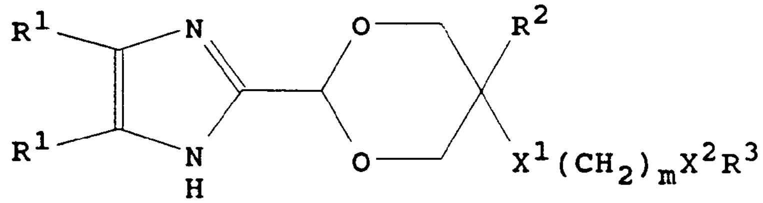

I

wherein the symbols $R^1$ are the same or different and each represents an optionally substituted phenyl group, $R^2$ represents an alkyl group, optionally substituted by a hydroxy group, $X^1$ represents a direct bond, an oxygen atom, or a group of the formula $-S(O)_n-$, $-CH_2O-$ or - $CH_2N(R^6)-$, wherein n is 0, 1 or 2 and $R^6$ represents a hydrogen atom, an alkyl group, an acyl group, an alkylsulphonyl group, or a group of the formula $-C(X^3)NR^7R^8$, wherein $X^3$ represents an oxygen or sulphur atom and $R^7$ and $R^8$ are the same or different and each represents a hydrogen atom, an alkyl group, or an optionally substituted phenyl group, m represents zero or an integer from 1 to 8, $X^2$ represents a direct bond, an oxygen atom, a group $-S(0)_p-$ wherein p is 0, 1 or 2, or a group of the formula $-C(0)NR^4-$, $-C(S)NR^4-$, $-OC(0)NR^4-$, $-OC(S)NR^4-$, $-NR^4C(0)-$ or $-NR^4C(S)-$, wherein $R^4$ represents a hydrogen atom or an alkyl group, and $R^3$ represents an optionally substituted heterocyclyl group, or a cycloalkyl group containing from 5 to 7 carbon atoms or, except when $X^1$ and $X^2$ simultaneously represent direct bonds, $R^3$ represents an optionally substituted phenyl group, or $R^3$ represents an amino, alkylamino, benzylamino, benzyl(alkyl)amino, dialkylamino, aminoalkyl, alkylaminoalkyl, benzylaminoalkyl, benzyl(alkyl)aminoalkyl or dialkylaminoalkyl group, or a cyclic imino group containing in the ring up to 7 carbon atoms, one or two of which can be in the form of a carbonyl group or groups, and the ring of which may be interrupted by a further nitrogen atom which may carry an alkyl or optionally substituted phenyl group, or a pharmaceutically acceptable salt thereof.

2. A compound according to claim 1 wherein the symbols $R^1$ are the same or different and each represents an optionally substituted phenyl group, $R^2$ represents an alkyl group, optionally substituted by a hydroxy group, $X^1$ represents a direct bond, an oxygen atom, or a group of the formula $-S(0)_n-$, $-CH_20-$ or $-CH_2N(R^6)-$, wherein n is 0, 1 or 2 and $R^6$ represents a hydrogen atom, an alkyl group, an acyl group, an alkylsulphonyl group, or a group of the formula $-C(X^3)NR^7R^8$, wherein $X^3$ represents an oxygen or sulphur atom and $R^7$ and $R^8$ are the same or different and each represents a hydrogen atom, an alkyl group, or an optionally substituted phenyl group, m represents zero or an integer from 1 to 8, $X^2$ represents a direct bond, an oxygen atom, a group $-S(0)_p-$ wherein p is 0, 1 or 2, or a group of the formula $-C(0)NR^4-$, $-C(S)NR^4-$, $-OC(0)NR^4-$, $-OC(S)NR^4-$, $-NR^4C(0)-$ or $-NR^4C(S)-$, wherein $R^4$ represents a hydrogen atom or an alkyl group, and, except when $X^1$ represents an oxygen atom or group $-CH_20-$, $R^3$ represents an optionally substitued heterocyclyl group, or $R^3$ represents a cycloalkyl group containing from 5 to 7 carbon atoms or, except when $X^1$ and $X^2$ simulaneously represent direct bonds, $R^3$ represents an optionally substituted phenyl group, or $R^3$ represents an amino, alkylamino, benzylamino, benzyl(alkyl)amino, dialkylamino, aminoalkyl, alkylaminoalkyl, benzylaminoalkyl, benzyl(alkyl)amonoalkyl or dialkylaminoalkyl group, or a cyclic imino group containing in the ring up to 7 carbon atoms, one or two of which can be in the form of a carbonyl group or groups, and the ring of which may be interrupted by a further nitrogen atom which may carry an alkyl or optionnally substitued phenyl group.

3. A compound according to claim 1 or 2 wherein n and p are identical.

4. A compound according to claim 1,2 or 3 wherein m is other than zero when $X^1$ and $X^2$ simultaneously represent groups $-S(0)_n-$ and $-S(0)_p-$ respectively.

5. A compound according to any one of the preceding claims wherein m is other than zero when neither of $X^1$ and $X^2$ represents a direct bond.

6. A compound according to any one of the preceding claims wherein m is other than zero when $X^2$ is other than a direct bond and simultaneously $R^3$ represents an amino, alkylamino, benzylamino, benzyl(alkyl)amino, or dialkylamino group.

7. A compound according to any one of the preceding claims wherein, unless otherwise specified, alkyl groups and moieties are straigth- or branched-chain and contain up to 6 carbon atoms; optionally substituted phenyl, benzyl and benzoyl groups are optionally substituted by one or more substitutents selected from halogen atoms and alkyl and alkoxy groups; acyl groups are alkanoyl groups or optionally substituted

benzoyl groups or their thio analogues; and an optionally substituted heterocyclyl group is a 5-, 6- or 7-membered ring containing 1 or 2 nitrogen atoms and optionally an oxygen or sulphur atom, optionally substituted by one or more substitutents selected from halogen atoms, amino groups, acylamino groups, alkyl and alkoxy groups, and optionally subtituted phenyl groups.

**8.** A compound according to any one of the preceding claims wherein the symbols $R^1$ are the same or different and each represents a phenyl group optionally carrying one or more substitutents selected from halogen atoms and alkyl and alkoxy group containing from 1 to 3 carbon atoms, $R^2$ represents a straight- or branched-chain alkyl group containing from 1 to 4 carbon atoms, optionally susbtituted by a hydroxy group, $X^1$ represents a direct bond, an oxygen atom, or a group $-S(0)_n-$ or $-CH_20-$ wherein n is 0, 1 or 2, m represents zero or an integer from 1 to 6, $X^2$ represents a direct bond, an oxygen atom, a group $-S(0)_p$ - wherein p is 0, 1 or 2, a group of the formula $-C(0)NR^4-$, $-C(S)NR^4-$, $-OC(0)NR^4-$, $-OC(S)NR^4-$, $-NR^4C(0)-$or $-NR^4C(S)-$, wherein $R^4$ represents a hydrogen atom or a straight- or branched-chain alkyl group containing from 1 to 4 carbon atoms, and, except when $X^1$ represents an oxygen atom or a group $-CH_20-$, $R^3$ represents an optionally substituted heterocyclyl group, or $R^3$ represents a cycloalkyl group containing from 5 to 7 carbon atoms or, except when $X^1$ and $X^2$ simultaneously represent direct bonds, $R^3$ represents a phenyl group optionally substituted by one or more substituents selected from halogen atoms and alkyl and alkoxy groups containing from 1 to 4 carbon atoms, or $R^3$ represents an amino group, a straight- or branched-chain alkylamino group containing from 1 to 4 carbon atoms, a straight- or branched-chain dialkylamino group containing from 1 to 4 carbon atoms in each alkyl group, or a cyclic imino group containing in the ring up to 7 carbon atoms, one or two of which can be in the form of a carbonyl group or groups.

**9.** A compound according to claim 1 hereinbefore identified as any one of compounds A to M.

**10.** A compound according to claim 1 hereinbefore identified as any one of compounds N to BH.

**11.** A process for the preparation of an imidazole derivative according to claim 1 which comprises:
(A) the replacement by hydrogen of the group $R^5$ of a compound of the general formula:

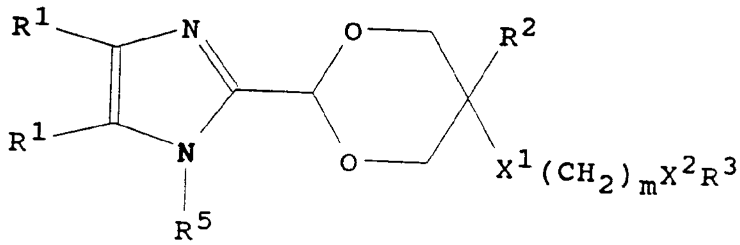

II

wherein $R^5$ represents a protecting group and the other symbols are as defined in claim 1;
(B) the reaction of a compound of the general formula:

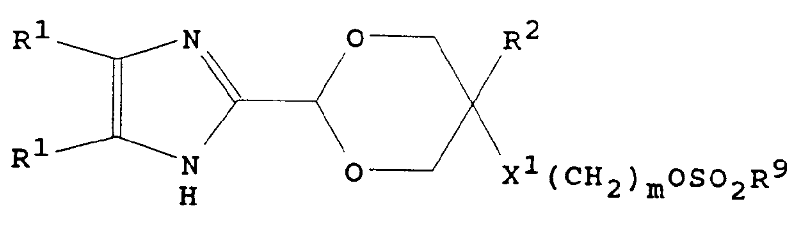

V

wherein $R^1$, $R^2$, $X^1$, $X^2$ and m are as defined in claim 1 and $R^9$ represents an alkyl or optionally substituted phenyl group, with a compound of the general formula:

$$HX^2R^3 \quad VI$$

wherein $X^2$ and $R^3$ are as defined in claim 1; (C) when $R^2$ in the imidazole derivative of general formula I represents a hydroxyalkyl group, the conversion to a hydroxyalkyl group of the protected hydroxyalkyl group $R^{10}$ of a compound of the general formula:

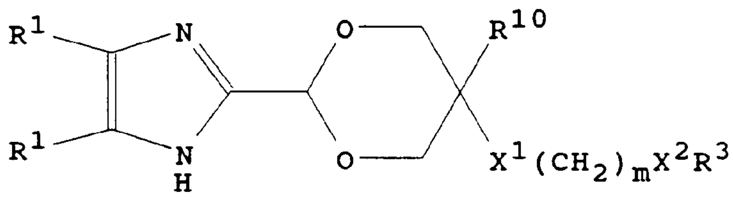

VII

wherein $R^{10}$ repesents a protected hydroxyalkyl group and the other symbols are as defined in claim 1;
(D) the reaction of a compound of the general formula:

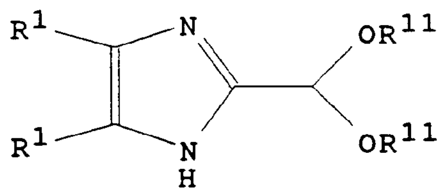

VIII

wherein $R^{11}$ represents an alkyl group and $R^1$ is as defined in claim 1, with a compound of the general formula:

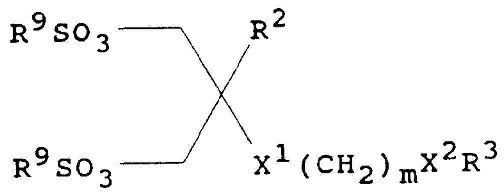

IX

wherein $R^9$ is as hereinbefore defined and the other symbols are as defined in claim 1; optionally followed by the conversion of an imidazole derivative of general formula I thus obtained into another imidazole derivative of general formula I; optionally followed by the conversion of an imidazole derivative thus obtained into a salt thereof.

**12.** A pharmaceutical composition which comprises an imidazole derivative according to claim 1 or a pharmaceutically acceptable salt thereof in association with a pharmaceutically acceptable carrier or coating.

**13.** A pharmaceutical composition useful in the treatment of a condition which can be ameliorated by an inhibitor of acyl coenzyme-A:cholesterol-0-acyl transferase or of the binding of $TxA_2$ to its receptors which comprises an effective amount of an imidazole derivative according to claim 1 or a pharmaceutically acceptable salt thereof.

**14.** A method of treating a human or animal patient suffering from, or subject to, a condition which can be ameliorated by the administration of an inhibitor of acyl coenzyme-A:cholesterol-0-acyl transferase or of the binding of $TxA_2$ to its receptors which comprises administering to the patient an effective amount of an imidazole derivative according to claim 1, or a pharmaceutically acceptable salt thereof, to secure an improvement in the condition of the patient.

European Patent Office

# EUROPEAN SEARCH REPORT

Application Number

EP 92 30 2680

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,A | EP-A-0 424 195 (RHONE-POULENC SANTE)<br>----- | | C07D405/04<br>A61K31/415<br>C07D405/14<br>A61K31/335 |
| | | | TECHNICAL FIELDS SEARCHED (Int. Cl.5) |
| | | | C07D<br>A61K |

The present search report has been drawn up for all claims

| Place of search | Date of completion of the search | Examiner |
|---|---|---|
| THE HAGUE | 24 JUNE 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P0401)